# EUROPÄISCHE PATENTANMELDUNG

(11) **EP 2 325 287 A1**
(43) Veröffentlichungstag der Anmeldung: **25.05.2011**
(21) Anmeldenummer: 10154449.2
(22) Anmeldetag: 23.02.2010
(51) Int. Cl.: C10J 3/66, F01K 23/06, F02C 3/28

(54) **Emissionsfreies Kraftwerk zur Erzeugung von elektrischer und mechanischer Energie**

(30) Priorität: 20.11.2009 EP 09176684
(71) Anmelder: RV Lizenz AG, 6300 Zug (CH)
(72) Erfinder: Rüdlinger, Mike, 8404 Winterthur (CH)
(74) Vertreter: Rentsch & Partner

(57) **Zusammenfassung**

Eine Anlage (1) zur Erzeugung von elektrischer und/oder mechanischer Energie (W2) durch thermisch-chemische Verwertung von kohlenstofFhaltigen Substanzen (A) umfasst einen ersten Anlagenteil (2) zur Verwertung der kohlenstoffhaltigen Substanzen (A), und einen weiteren Anlagenteil (7) zur Erzeugung von elektrischer und/oder mechanischer Energie (W2). Der erste Anlagenteil (2) umfasst eine erste Stufe (3) zur Durchführung einer Pyrolyse der kohlenstoffhaltigen Substanzen (A) zu Pyrolysekoks (D) und Pyrolysegas (E), eine zweite Stufe (4) zur Durchführung einer Vergasung des Pyrolysekokses (D) zu Synthesegas (S) und Reststoffen (H, K) und eine dritte Stufe (5) zur Durchführung einer Umwandlung des Synthesegases (S) in kohlenstoffhaltige Betriebsstoffe (Q). Alle drei Stufen (3, 4, 5) des ersten Anlagenteils (2) sind druckfest geschlossen und bilden einen im Wesentlichen geschlossenen Kreislauf. Eine Transportleitung für das Pyrolysegas (E) verbindet den ersten Anlagenteil (3) druckfest mit dem zweiten Anlagenteil (4) und/oder mit dem dritten Anlagenteil (5). Eine Transportleitung für das Synthesegas (S) verbindet den zweiten Anlagenteil (4) druckfest mit dem dritten Anlagenteil (5) und/oder mit dem ersten Anlagenteil (3). Eine Transportleitung für das Rücklaufgas (R) verbindet den dritten Anlagenteil (5) druckfest mit dem ersten Anlagenteil (3) und/oder mit dem zweiten Anlagenteil (4). In dem weiteren Anlagenteil (7) ist mindestens eine Antriebsvorrichtung (71) vorgesehen zur Produktion (74) von elektrischer und/oder mechanischer Energie (W2) aus den kohlenstoffhaltigen Betriebsstoffen (Q).

## Beschreibung

### Technisches Gebiet

Die Erfindung betrifft mit kohlenwasserstoffhaltigen Materialien betriebene Kraftwerksanlagen zur Erzeugung von elektrischem Strom und entsprechende Verfahren.

### Stand der Technik

Schon länger ist nun bekannt, dass Kohlendioxid sehr negative Auswirkungen auf das Klimagleichgewicht der Erde hat und stark zur menschengemachten Klimaerwärmung beiträgt. Die Vermeidung von Kohlendioxidemissionen ist daher sehr wünschenswert, insbesondere bei der Erzeugung von elektrischer und/oder mechanischer Energie aus fossilen Brennstoffen.

Bei Verwendung kohlenstoffhaltiger Substanzen als Betriebsstoffe für herkömmliche Kraftwerksanlagen ist Kohlendioxid ein unvermeidliches Nebenprodukt des Energiegewinnung. Ein Herausfiltern von Kohlendioxid aus entstehenden Verbrennungsabgasen ist in der Regel mit vernünftigem energetischem Aufwand nur schwer möglich. Für den grossindustriellen Massstab werden Systeme gestestet, in welchen das Kohlendioxid beispielsweise in amin-basierten Lösungsmitteln aufgefangen wird. Solche Systeme sind jedoch aufwendig und kompliziert, und für kleinere Anlagen nicht praktikabel.

Andere Energiequellen ohne Kohlendioxidemissionen wie beispielsweise Wasserkraft und Nuklearenergie weisen andere Probleme auf. Neuere Anlagen zur Nutzung alternativer Energiequellen wie Windkraft, Solarenergie und kohlendioxidneutraler Biomasse weisen zu geringe Kapazitäten zur Deckung des stetig wachsenden Energiebedarfs auf. Wetterabhängige Energiequellen können zudem oft die notwendigen Leistungskapazitäten nicht bedingungslos sicherstellen. Anlagen zur emissionsarmen, effizienten, flexiblen und leicht skalierbaren Energieproduktion, insbesondere von elektrischer Energie, sind deshalb Gegenstand grosser Forschungsbemühungen.

Es sind aus dem Stand der Technik verschiedene Typen von Verfahren und Anlagen bekannt, mit denen aus festen, flüssigen und gasförmigen kohlenstoffhaltigen Substanzen Gasgemische mit Kohlenmonoxid und Wasserstoff hergestellt werden können, die anschliessend als Synthesegas für chemische Synthesen oder als Betriebsstoffe für Kraftwerksanlagen verwendet werden können. Zur Herstellung von Kohlenmonoxid-WasserstoffSynthesegasen aus festem Kohlenstoff wird dieser mit Hilfe von Sauerstoff, Kohlendioxid oder Wasser zu Synthesegas vergast:

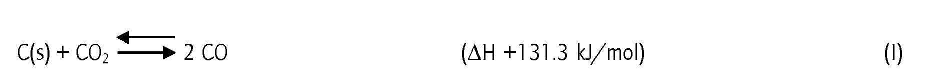

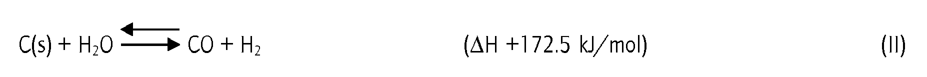

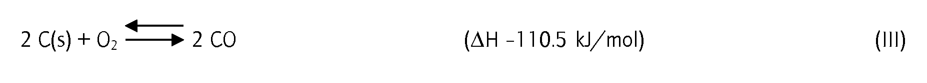

Das Verhältnis zwischen Kohlenmonoxid und Wasserstoff ist durch die sogenannte Wassergas-Stift-Reaktion IV gegeben:

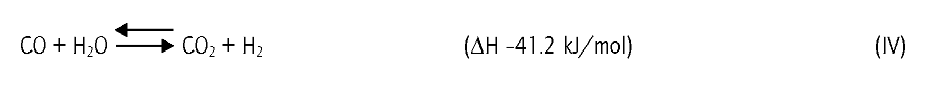

Die für den Ablauf der endothermen Reaktionen I und II notwendige thermische Energie kann beispielsweise aus einer partiellen Verbrennung des festen Kohlenstoffs in der Reaktion III stammen, oder extern zugeführt werden.

Die bekannten Verfahren zur Herstellung von Synthesegas sind darauf ausgerichtet, aus kostengünstiger fossiler Kohle Synthesegas für die chemische Industrie herzustellen. Andere solche Verfahren sind primär darauf ausgerichtet, aus festen kohlenstoffhaltigen Substanzen wie beispielsweise fossiler Kohle, Biomasse, oder heterogenen Gemischen wie beispielsweise brennbarem Abfall leichter handhabbaren gasförmigen Brennstoff herzustellen. Mit diesem können dann zum Beispiel Gasturbinen zur Erzeugung elektrischer und/oder mechanischer Energie betrieben werden. Jedoch wird bei diesen Verfahren ein Teil der im festen Ausgangmaterial gespeicherten chemischen Energie bei der Umwandlung, entweder bei der Koksherstellung oder bei der Gasherstellung, verbraucht und entsprechend Kohlendioxid freigesetzt, was die Kohlendioxideffizienz senkt. Ein Nachteil der bekannten Verfahren ist neben der geringen Effizienz der komplizierte Aufbau, insbesondere bei Anlagen, bei welchem Koks im Wirbelstrom oder Flugstrom vergast wird. Solche Verfahren benötigen zudem eine aufwendige Steuerung.

### Aufgabe der Erfindung

Aufgabe der Erfindung ist es, eine emissionsfreie Kraftwerksanlage der eingangs erwähnten Art zur Verfügung zu stellen, welche die oben erwähnten und andere Nachteile nicht aufweisen.

Eine andere Aufgabe der Erfindung ist es, eine Kraftwerksanlage zur Verfügung zu stellen, welche es erlaubt, elektrische und/oder mechanische Energie effizient und bedarfsgerecht in einem breiten Leistungsband bereitzustellen.

Insbesondere soll eine solche erfindungsgemässe Kraftwerksanlage Energie speichern können und bei erhöhtem Leistungsbedarf diese gespeicherte Energie als elektrische und/oder mechanische Energie wieder abgeben können.

Eine solche Kraftwerksanlage soll vorzugsweise einen breiten Bereich an festen, flüssigen und gasförmigen kohlenstoffhaltigen Substanzen und Gemischen, insbesondere Kohle, Biomasse und anderen heterogenen Materialien zur Energieerzeugung verwerten können.

Noch eine Aufgabe der Erfindung ist es, eine Kraftwerksanlage zur Verfügung zu stellen, mit denen schwierig zu verwertende erdölhaltige Substanzen wie beispielsweise Ölschiefer, Ölsand oder Ölschlamm emissionsfrei zur Energieproduktion nutzen zu können.

Diese und andere Aufgaben werden gelöst durch eine erfindungsgemässe Kraftwerksanlage und ein erfindungsgemässes Verfahren gemäss den unabhängigen Ansprüchen. Weitere vorteilhafte Ausführungsformen sind in den abhängigen Ansprüchen gegeben.

### Darstellung der Erfindung

Eine erfindungsgemässe zur Erzeugung von elektrischer und/oder mechanischer Energie durch thermisch-chemische Verwertung von kohlenstoffhaltigen Substanzen umfasst ersten Anlagenteil zur Verwertung der kohlenstoffhaltigen Substanzen, und einen weiteren Anlagenteil zur Erzeugung von elektrischer und/oder mechanischer Energie. Der erste Anlagenteil umfasst eine erste Stufe zur Durchführung einer Pyrolyse der kohlenstoffhaltigen Substanzen zu Pyrolysekoks und Pyrolysegas, eine zweite Stufe zur Durchführung einer Vergasung des Pyrolysekokses zu Synthesegas und Reststoffen und eine dritte Stufe zur Durchführung einer Umwandlung des Synthesegases in kohlenstoffhaltige Betriebsstoffe. Alle drei Stufen des ersten Anlagenteils sind druckfest geschlossen und bilden einen im Wesentlichen geschlossenen Kreislauf. Eine Transportleitung für das Pyrolysegas verbindet den ersten Anlagenteil druckfest mit dem zweiten Anlagenteil und/oder mit dem dritten Anlagenteil. Eine Transportleitung für das Synthesegas verbindet den zweiten Anlagenteil druckfest mit dem dritten Anlagenteil und/oder mit dem ersten Anlagenteil. Eine Transportleitung für das Rücklaufgas verbindet den dritten Anlagenteil druckfest mit dem ersten Anlagenteil und/oder mit dem zweiten Anlagenteil. In dem weiteren Anlagenteil ist mindestens eine Antriebsvorrichtung vorgesehen zur Produktion von elektrischer und/oder mechanischer Energie aus den kohlenstoffhaltigen Betriebsstoffen.

In einer vorteilhaften Ausführungsform einer solchen erfindungsgemässen Kraftwerksanlage bezieht die Antriebsvorrichtung die zum Betrieb notwendige Energie aus der Oxidation der kohlenstoffhaltigen Betriebsstoffe zu einem Produktgas, das im wesentlichen aus Kohlendioxid und Wasser besteht. Weiter umfasst die Antriebsvorrichtung eine Vorrichtung zur Verdichtung und/oder Kondensation des Produktgases. Die Antriebsvorrichtung kann als Brennstoffzelle oder als Wärmekraftmaschine ausgestaltet sein. In einer besonders vorteilhaften Variante ist die Antriebsvorrichtung mit reinem Sauerstoff als Oxidationsmittel betreibbar.

In einer weiteren Ausführungsform einer erfindungsgemässen Kraftwerksanlage ist ein Wärmetauscher zur Abkühlung des Produktgasstromes vor und/oder nach der Vorrichtung zur Verdichtung und/oder Kondensation des Produktgases vorgesehen.

In noch einer weiteren Ausführungsform einer erfindungsgemässen Kraftwerksanlage ist eine Vorrichtung zur Kondensation und/oder Abscheidung von Wasser aus dem Produktgas vorgesehen. Dies reduziert die Menge des verbleibenden Restgases.

Eine andere Variante einer erfindungsgemässen Kraftwerksanlage umfasst einen Speicher zum Auffangen des Produktgases bzw. Restgases nach Verdichtung und/oder Kondensation des Produktgases.

Zur Rückführung der Produktgase oder Restgase in eine der drei Stufen kann des ersten Anlagenteils einer erfindungsgemässen Kraftwerksanlage kann eine Transportleitung vorgesehen sein.

In einer anderen vorteilhaften Ausführungsform einer der vorgenannten erfindungsgemässen Kraftwerksanlage ist die Antriebsvorrichtung als Verbrennungskraftmaschine ausgestaltet, mit mindestens einer Brennkammer zur Verbrennung von flüssigem oder gasförmigem Betriebsstoff mit Sauerstoff, mit Mitteln zur Umsetzung des entstehenden Gasdrucks bzw. Gasvolumens in mechanische Arbeit, mit einer Zufuhrvorrichtung zum Einbringen von Sauerstoff in die Brennkammer, und mit einer Entlüftungsvorrichtung zur Entfernung der Produktgase aus der Brennkammer. In einer besonders vorteilhaften Variante einer solchen erfindungsgemässen Kraftwerksanlage ist bei der Antriebsvorrichtung eine Zufuhrvorrichtung zum Einbringen von Wasser und/oder Wasserdampf in die Brennkammer und/oder in den Produktgasstrom nach dem Austritt aus der Brennkammer vorgesehen. Die Antriebsvorrichtung kann beispielsweise eine Turbinenvorrichtung umfassen, welche mit dem Produktgasstrom betrieben wird.

Eine erfindungsgemässe Kraftwerksanlage weist vorteilhaft im Kreislauf des ersten Anlagenteils mindestens einen Wärmetauscher zur Erhitzung von Wasserdampf und/oder Erzeugung von Wasserdampf auf, sowie einen weiteren Anlagenteil mit einer weiteren Antriebs-vorrichtung, bevorzugt eine Dampfturbine, zur Produktion von elektrischer und/oder mechanischer Energie aus erhitztem Wasserdampf. Bevorzugt wird die genannte weitere Antriebsvorrichtung mit Wasserdampf betrieben, welcher zuvor in einem Wärmetauscher des ersten Anlagenteils überhitzt worden ist.

Eine andere erfindungsgemässe Kraftwerksanlage zur Erzeugung von elektrischer und/oder mechanischer Energie durch thermisch-chemische Verwertung von kohlenstoffhaltigen Substanzen, weist einen ersten Anlagenteil zur Verwertung der kohlenstoffhaltigen Substanzen, und einen weiteren Anlagenteil zur Erzeugung von elektrischer und/oder mechanischer Energie. Der erste Anlagenteil umfasst eine erste Stufe zur Durchführung einer Pyrolyse der kohlenstoffhaltigen Substanzen zu Pyrolysekoks und Pyrolysegas, eine zweite Stufe zur Durchführung einer Vergasung des Pyrolysekokses zu Synthesegas und Reststoffen und eine dritte Stufe zur Durchführung einer Umwandlung des Synthesegases in kohlenstoffhaltige Betriebsstoffe. Alle drei Stufen des ersten Anlagenteils sind druckfest geschlossen und bilden einen im Wesentlichen geschlossenen Kreislauf. Eine Transportleitung für das Pyrolysegas verbindet den ersten Anlagenteil druckfest mit dem zweiten Anlagenteil und/oder mit dem dritten Anlagenteil. Eine Transportleitung für das Synthesegas verbindet den zweiten Anlagenteil druckfest mit dem dritten Anlagenteil und/oder mit dem ersten Anlagenteil. Eine Transportleitung für das Rücklaufgas verbindet den dritten Anlagenteil druckfest mit dem ersten Anlagenteil und/oder mit dem zweiten Anlagenteil. Im Kreislauf des ersten Anlagenteils ist mindestens ein Wärmetauscher zur Erhitzung von Wasserdampf und/oder Erzeugung von Wasserdampf vorgesehen. Im weiteren Anlagenteil wiederum ist eine Antriebsvorrichtung vorgesehen, bevorzugt eine Dampfturbine, zur Erzeugung von elektrischer und/oder mechanischer Energie aus dem erhitzten Wasserdampf. Vorteilsweise ist die Antriebsvorrichtung dazu eingerichtet, mit Wasserdampf betrieben zu werden, welcher zuvor in einem Wärmetauscher des ersten Anlagenteils überhitzt worden ist.

Bei einem erfindungsgemässen Verfahren zur Erzeugung elektrischer und/oder mechanischer Energie durch thermisch-chemische Verwertung von kohlenstoffhaltigen Substanzen werden in einer ersten Stufe die kohlenstoffhaltigen Substanzen zugeführt und pyrolysiert, wobei Pyrolysekoks und Pyrolysegas entstehen. In einer zweiten Stufe wird der Pyrolysekoks aus der ersten Stufe vergast, wobei Synthesegas entsteht, und Schlacke und andere Reststoffe übrig bleiben und abgeführt werden. In einer dritten Stufe wird das Synthesegas aus der zweiten Stufe in kohlenstoffhaltige Betriebsstoffe umgewandelt. Diese Betriebsstoffe werden abgeführt. Überschüssiges Rücklaufgas aus der dritten Stufe wird in die erste Stufe und/oder die zweite Stufe geleitet. Die drei Stufen bilden einen geschlossenen Kreislauf. Durch Oxidation der kohlenstoffhaltigen Betriebsstoffe aus der dritten Stufe zu einem Produktgas im Wesentlichen bestehend aus Kohlendioxid und Wasser wird elektrische und/oder mechanische Energie erzeugt. Als Oxidationsmittel wird dabei vorteilsweise reiner Sauerstoff verwendet. Aus den Produktgasen kann Wasser auskondensiert und/oder abgeschieden werden.

In einer vorteilhaften Variante eines solchen erfindungsgemässen Verfahrens wird mindestens ein Teil der Produktgase der Antriebsvorrichtung wieder in die erste Stufe und/oder die zweite Stufe und/oder die dritte Stufe eingespeist.

Entlang des Kreislaufs liegt vorteilsweise ein Druckgefälle vor. Die Zufuhr der Wärmeenergie für die Pyrolysereaktionen in der ersten Stufe erfolgt bevorzugt zum Teil oder vollständig durch Rückleiten eines Teils des heissen Synthesegases aus der zweiten Stufe in die erste Stufe, und/oder durch partielle Oxidation des Ausgangsmaterials und des entstehenden Pyrolysekokses.

Die erste Stufe kann bei einer Temperatur zwischen 300 und 800 °C durchgeführt werden, bevorzugt zwischen 450 und 700 °C, und besonders bevorzugt zwischen 500 und 600 °C. Die zweite Stufe kann bei einer Temperatur zwischen 600 und 1600 °C, bevorzugt zwischen 700 und 1400 °C, und besonders bevorzugt zwischen 850 und 1000 °C, durchgeführt werden. Der Druck in der erste Stufe und/oder der zweiten Stufe beträgt vorteilhaft 10 und 60 bar, vorzugweise zwischen 10 und 25 bar.

Für die Vergasungsreaktion in der zweiten Stufe wird vorteilhaft Sauerstoff und/oder Wasserdampf und/oder Kohlendioxid verwendet. Die für die Vergasungsreaktion notwendige thermische Energie kann zum Teil oder vollständig von aussen zugeführt werden, beispielsweise durch Heizvorrichtungen und/oder Wärmetauscher; und/oder durch Oxidation eines Teils des Pyrolysekokses mit einem Oxidationsmittel, insbesondere Sauerstoff, erzeugt werden.

In einer besonders vorteilhaften Variante eines erfindungsgemässen Verfahrens wird in einem Wärmetauscher das Synthesegas abgekühlt, wobei überhitzter Wasserdampf entsteht, aus welchem mit einer Wärmekraftmaschine elektrische und/oder mechanische Energie produziert wird.

Bei einem weiteren erfindungsgemässen Verfahren zur Erzeugung elektrischer und/oder mechanischer Energie durch thermisch-chemische Verwertung von kohlenstoffhaltigen Substanzen werden in einer ersten Stufe die kohlenstoffhaltigen Substanzen zugeführt und pyrolysiert, wobei Pyrolysekoks und Pyrolysegas entstehen. In einer zweiten Stufe wird der Pyrolysekoks aus der ersten Stufe vergast, wobei Synthesegas entsteht, und Schlacke und andere Reststoffe übrig bleiben und abgeführt werden. In einer dritten Stufe wird das Synthesegas aus der zweiten Stufe in kohlenstoffhaltige Betriebsstoffe umgewandelt. Diese Betriebsstoffe werden abgeführt. Überschüssiges Rücklaufgas aus der dritten Stufe wird in die erste Stufe und/oder die zweite Stufe geleitet. Die drei Stufen bilden einen geschlossenen Kreislauf. In einem Wärmetauscher wird das Synthesegas abgekühlt, wobei überhitzter Wasserdampf entsteht, aus welchem mit einer Wärmekraftmaschine elektrische und/oder mechanische Energie produziert wird.

### Kurze Beschreibung der Zeichnungen

Im Folgenden wird die erfindungsgemässe Vorrichtung anhand von Zeichnungen erläutert. Diese zeigen lediglich Ausführungsbeispiele des Erfindungsgegenstands.
- Figur 1: zeigt (a) schematisch eine erfindungsgemässe Kraftwerksanlage, und (b) eine solche Anlage mit einem vom Verwertungsanlagenteil und Grundlastanlagen- teil räumlich getrenntem Spitzenlastanlagenteil.

- Figur 1A: zeigt schematisch das Leistungsprofil (a) einer herkömmlichen thermischen Kraftwerksanlage, (b), (c) von erfindungsgemässen Kraftwerksanlagen, und (d) Spitzenlast- und Grundlastprofil einer erfindungsgemässen Kraftwerksanlage.
- Figuren 2 bis 6: zeigen schematisch verschiedene mögliche Ausführungsbeispiele von Verwer- tungsanlagen in einer erfindungsgemässen Kraftwerksanlage.
- Figur 8: zeigt schematisch ein allgemeines Ausführungsbeispiel einer erfindungsgemäs- sen Kraftwerksanlage mit einer Grundlast-Generatoranlage.
- Figur 9: zeigt schematisch eine mögliche Ausführungsform einer erfindungsgemässen Kraftwerksanlage mit einer Spitzenlast-Generatoranlage.
- Figur 10: zeigt schematisch eine mögliche Ausführungsform einer erfindungsgemässen Kraftwerksanlage mit einer Grundlast-Generatoranlage und einer Spitzenlast- Generatoranlage.
- Figur 11: zeigt schematisch zwei Ausführungsformen einer als Verbrennungskraftma- schine ausgestalteten Antriebsvorrichtung einer Spitzenlast-Generatoranlage.
- Figur 12: zeigt schematisch eine als kombinierte Gas/Dampfturbine ausgestaltete An- triebsvorrichtung einer Spitzenlast-Generatoranlage.

### Ausführung der Erfindung

Die im Folgenden gegebenen Beispiele werden zur besseren Veranschaulichung der vorliegenden Erfindung gegeben, sind jedoch nicht dazu geeignet, die Erfindung auf die hierin offenbarten Merkmale zu beschränken.

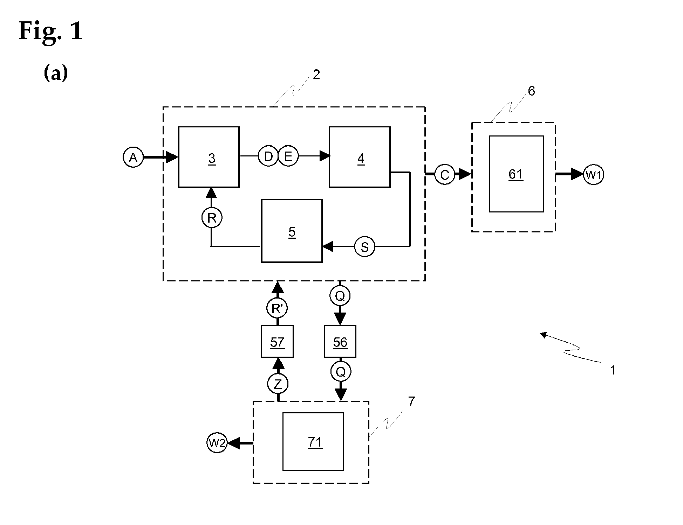

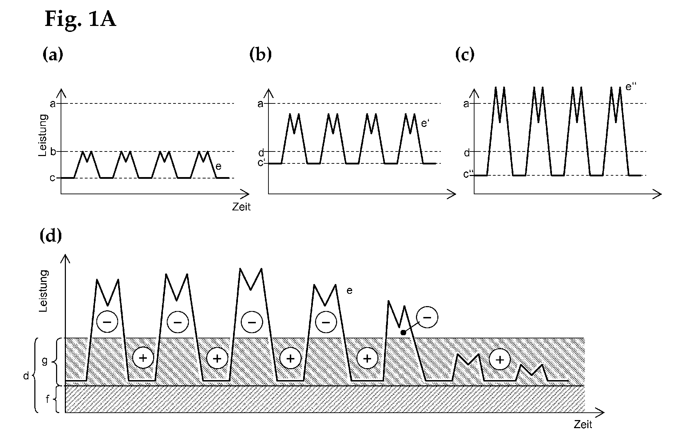

Figur 1 (a) zeigt schematisch eine mögliche Ausführungsform einer erfindungsgemässen Kraftwerksanlage 1 zur Erzeugung vom elektrischer und/oder mechanischer Energie, mit einem ersten Anlagenteil 2 zur thermisch-chemischen Verwertung von kohlenstoffhaltigen Substanzen A zu flüssigen und/oder gasförmigen Betriebsstoffen Q (chemische Energie) und Prozessdampf B, C (thermische Energie), einem zweiten Anlagenteil 6 zur Erzeugung von elektrischer und/oder mechanischer Energie aus thermischer Energie in Form von Prozessdampf C, und einen dritten Anlagenteil 7 zur Erzeugung von elektrischer und/oder mechanischer Energie aus chemischer Energie in Form der Betriebsstoffe Q.

Im Verwertungs-Anlagenteil 2 werden in einer ersten Stufe 3 die kohlenstoffhaltigen Substanzen zugeführt und pyrolysiert, wobei Pyrolysekoks D und Pyrolysegas E entstehen. In einer zweiten Stufe 4 wird der Pyrolysekoks D aus der ersten Stufe vergast, wobei Synthesegas S entsteht, und Schlacke und andere Reststoffe übrig bleiben. In einer dritten Stufe 5 wird das Synthesegas S aus der zweiten Stufe in kohlenwasserstoffbasierte flüssige und/oder gasförmige Betriebsstoffe Q umgewandelt. Alle drei Stufen sind druckfest geschlossen und bilden einen im Wesentlichen geschlossenen Kreislauf. Mit eine solchen Verwertungs-Anlage 2 können feste, flüssige oder gasförmige Substanzen effizient in gasförmige oder flüssige Betriebsstoffe Q umgewandelt werden. Zusätzlich erzeugt die Anlage 2 thermische Energie in Form von Prozessdampf C.

Im Verwertungsverfahren anfallende thermische Energie kann in Form von Prozessdampf C aus dem ersten Anlagenteil 2 entnommen und im zweiten Anlagenteil 6 zur Erzeugung elektrischer und/oder mechanischer Energie W1 mittels einer geeigneten Antriebsvorrichtung 61 verwendet, beispielsweise mittels einer Dampfturbine. Während eines konstanten Betriebs der Verwertungsanlage 2 wird so eine gewisse Grundleistung erzeugt. Dieser zweite Anlagenteil 6 ist optionaler Bestandteil einer erfindungsgemässen Kraftwerksanlage.

Die in der Synthese-Stufe 5 erzeugten kohlenwasserstoffhaltigen Betriebsstoffe werden zwischengelagert 56, in Tanks oder Druckspeichern. Aus diesen Speichern 56 werden die Betriebsstoffe Q entnommen, und im dritten Anlagenteil 7 mit einer geeigneten Antriebsvorrichtung 71 in elektrische und/oder mechanische Energie W2 umgewandelt. Dies kann beispielsweise mittels einer Wärmekraftmaschine oder einer Brennstoffzellenvorrichtung geschehen. Kohlendioxidhaltiges Produktgas Z aus dem dritten Anlagenteil 7 wird wieder in den Verwertungs-Anlagenteil 2 zurückgeführt. Gegebenenfalls kann ein Zwischenspeicher 57 vorgesehen sein.

Der dritten Anlagenteil 7 bietet den Vorteil, dass die von der erfindungsgemässen Anlage produzierte Energieleistung in sehr kurzer Zeit an den gerade erforderlichen Bedarf angepasst werden kann. Die chemischen Betriebsstoffe Q dienen dabei als Energie-Zwischenspeicher. Während einer Stromverbrauchs-Spitze kann dann eine geeignet ausgestaltete Vorrichtung 61, beispielsweise eine mit den Betriebsstoffen Q betriebene Gasturbine und/oder Dampfturbine, sehr schnell in Betrieb genommen werden. Die Spitzenleistung der Anlage 1 kann aufgrund der Energiespeicherkapazität die thermische Grundleistung der Anlage kurzfristig übersteigen.

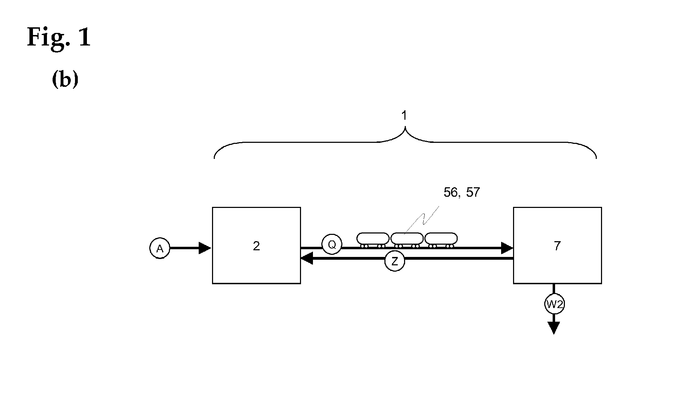

Der dritte Anlagenteil 7 kann zusammen mit dem Verwertungsanlagenteil 2 am gleichen Ort installiert sein. Alternativ ist es auch möglich, wie in Figur 1(b) dargestellt, das der dritte Anlagenteil 7 eines Gesamtsystems 1 vom ersten Anlagenteil räumlich getrennt angeordnet ist. Der Transport der Betriebsstoffe Q und der Produktgase Z kann beispielsweise per Eisenbahn oder Pipeline erfolgen, wobei in einem solchen Fall die Transportvorrichtung (Tankwagen, Pipeline) auch als Speicher 56, 57 dient. Da der Transport von chemischer Energie in Form von Betriebstoffen Q über grosse Distanzen wesentlich effizienter ist als die Übertragung von elektrischer Energie, kann der Standort des Spitzenlast-Anlagenteils 7 einer erfindungsgemässen Kraftwerksanlage 1 dort gewählt werden, wo der entsprechende Bedarf anfällt, während der Verwertungsanlagenteil 2 vorteilsweise dort aufgebaut ist, wo die kohlenstoffhaltigen Ausgangsstoffe anfallen.

Der Unterschied des Leistungsspektrums einer erfindungsgemässen Kraftwerksanlage 1 im Vergleich zu einem herkömmlichen, mit kohlenstoffhaltigen Betriebstoffen betriebenen Kraftwerk wird in den Figuren 1A(a) bis (d) genauer erläutert.

Figur 1A(a) zeigt schematisch das Leistungsprofil eines herkömmlichen thermischen Kraftwerks. Die vertikale Achse stellt dabei die Leistung dar, und die horizontale Achse die Zeit. Das Kraftwerk weist einen zugefügten Wärmeinhalt a auf, also die im Brennstoff als chemische Energie enthaltene Wärmenergie bzw. -Leistung, und eine effektive thermische Leistung b, also die effektiv in elektrische oder mechanische Energie umsetzbare Wärmeenergie pro Zeiteinheit. Der Bedarf an elektrischer Leistung e in einem üblichen Stromnetz variiert sowohl während des Tages als auch während der Woche. Um mit einem Kraftwerk neben der Grundlast c auch die Spitzenlasten abdecken zu können, muss die Gesamtnennleistung einer solchen Kraftwerksanlage auf die Spitzenlast ausgerichtet werden. Das heisst, die Dimensionierung der Anlage ist wegen der benötigten Spitzenleistung grösser als es aufgrund der durchschnittlichen Gesamtleistung eigentlich nötig wäre.

In einer erfindungsgemässen Kraftwerksanlage gemäss Figur 1(a) oder (b) ist dies hingegen nicht notwendig. Eine solche Kraftwerksanlage 1 setzt in der Verwertungsanlage 2 einen konstanten Teil der in Form der kohlenstoffhaltigen Substanzen A zugeführten chemischen Energie in thermische Energie in Form von Wasserdampf C um, welcher dann beispielsweise mit einer Dampfturbine 61 des Grundlast-Anlagenteils 6 in elektrische Energie f umgewandelt wird. Ein weiterer Anteil der in Form der kohlenstoffhaltigen Substanzen A zugeführten chemischen Energie wird in der Synthesestufe 5 der Verwertungsanlage 2 mit einer konstanten Produktionsleistung g in chemische Energie in Form von hochwertigen kohlenstoffhaltigen Betriebstoffen Q umgewandelt, beispielsweise dieselähnliche Produkte oder gasförmige Produkte wie Propan. Diese Betriebstoffe können in beliebiger Menge gespeichert 56 werden, und/oder wie in Figur 1 (b) ausgeführt über kurze oder weitere Strecken transportiert werden.

Figur 1A(d) zeigt schematisch das Profil der Gesamtleistung e einer erfindungsgemässen Kraftwerksanlage über den Verlauf einer Woche. Der Spitzenlast-Anlagenteil 7 erzeugt nun während des Spitzenlast-Bedarfs während der Werktage elektrische Energie aus den chemischen Betriebsstoffen Q, welche dann zu einem entsprechend hohen Preis in ein Energienetz eingespeist werden kann. Der Bedarf an chemischen Betriebstoffen Q übersteigt dabei die Produktionsleistung g der Verwertungsanlage 2 wesentlich, was mit (-) markiert ist. Dieser überdurchschnittliche Verbrauch wird dem Betriebsstoffspeicher 56 entnommen. Während der Nacht und am Wochenende sinkt der Bedarf stark ab, und die Produktionsleistung g übersteigt den Bedarf e, was mit (+) markiert ist. Als Folge davon wird der Betriebsstoffspeicher 56 wieder aufgefüllt.

Während der Grundlastperioden kann der dritten Anlagenteil 7 auf ein minimales Leistungsniveau heruntergefahren werden, wie in Figur 1A(d) dargestellt, oder der Anlagenteil 7 wird vollständig ausser Betrieb genommen, so dass die Grundlast c vollständig vom zweiten Anlagenteil 6 gedeckt wird.

Eine erfindungsgemässe Kraftwerksanlage hat also den wesentlichen Vorteil, dass nur ein Teil f der konstanten effektiven Leistung d in Form von thermischer Leistung anfällt, die wie bei einem herkömmlichen Kraftwerk umgehend in elektrische und/oder mechanische Energie umgesetzt werden muss. Dieser Teil f kann dazu genutzt werden um die Leistung für den Grundlast-Sockel c zu liefern. Ein anderer Teil g der effektiven Leistung d wird hingegen in Form von Betriebstoffen Q im Speicher 56 zwischengelagert. Der die thermische Leistung des Grundlast-Anlagenteils 6 übersteigende Bedarf (e - f) kann dann vom dritten Anlagenteil 7 aus dem Betriebstoffspeicher 56 gedeckt werden. Dies erlaubt es, eine erfindungsgemässe Kraftwerksanlage so auszulegen, dass die effektive Leistung d, zusammengesetzt aus thermischer Leistung f des zweiten Anlagenteils 6 und Produktionsleistung der Synthesestufe 5, dem gemittelten, durchschnittlichen Bedarf entspricht, wie in Figur 1A(b) gezeigt. Dies führt dann dazu, dass bei einer erfindungsgemässen Kraftwerksanlage mit gleicher effektiver thermischer Leistung d wie die thermische Leistung b einer herkömmlichen Kraftwerksanlage eine vergleichsweise höhere Grundlast-Leistung c' und eine höhere Spitzenlast-Leistung erreicht, wobei die Spitzenleistung die effektive thermische Leistung d kurzfristig erheblich übersteigen kann.

Umgekehrt betrachtet kann eine erfindungsgemässe Kraftwerksanlage 1 mit einer wesentlich kleineren installierten thermischen Leistung ausgelegt werden, um ein bestimmtes Bedarfsprofil abdecken zu können, beispielsweise 75% oder 50% der thermischen Leistung eines vergleichbaren herkömmlichen Kraftwerks. Dies führt zu wesentlich niedrigeren Investitionskosten.

Ein erfindungsgemässes Kraftwerk kann so ausgelegt und optimiert werden, dass die direkt aus thermischer Energie erzeugte Leistung f zu Gunsten der aus den Betriebsstoffen Q erzeugten Leistung g reduziert ist. Eine solche Variante ist in Figur 1A(c) dargestellt. Eine solche erfindungsgemässe Anlage kann bei Deckung eines reduzierten Grundlast-Sockels c" eine wesentlich höhere Energiemenge speichern. Die entsprechende gespeicherte Energie kann schliesslich zur Erzeugung von Spitzenlast-Leistung e" verwendet werden, die dann zu einem höheren Preis verkauft werden kann.

Es ist gegebenenfalls möglich, eine erfindungsgemässe Kraftwerksanlage soweit auf die flexible Erzeugung von Spitzenlast-Energie zu optimieren, dass die Grundlastleistung des zweiten Anlagenteils 6 minimal ist, und eventuell nur noch ausreicht, um den internen Energiebedarf der Kraftwerksanlage zu decken.

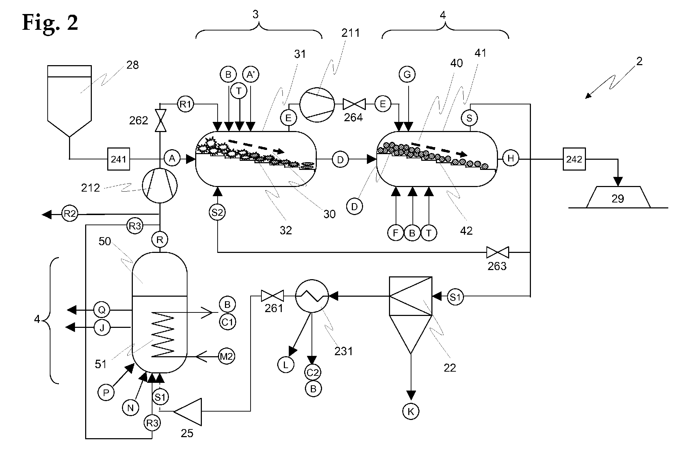

Eine erste Variante einer Verwertungsanlage 2 einer erfindungsgemässen Kraftwerksanlage 1 ist in Figur 2 schematisch dargestellt. Die Verwertungs-Anlage 2 enthält drei Stufen 3, 4, 5 zur Durchführung der drei Schritte des Verwertungsverfahrens, die so zu einem geschlossenen Kreislauf verbunden sind, dass sie einen zyklischen Gasstrom erlauben. In der ersten Stufe 3 (Pyrolyse-Stufe) wird kohlenstoffhaltiges Ausgangsmaterial unter Druck pyrolysiert, wodurch Pyrolysekoks und Pyrolysegase entstehen. In einer zweiten Stufe 4 (Vergasungs-Stufe) wird Pyrolysekoks zu Synthesegas vergast, welches schliesslich in einer dritten Stufe 5 (Synthese-Stufe) zu den Betriebstoffen Q reagiert wird. Die zu verarbeitenden kohlenstoffhaltigen Ausgangsmaterialien A werden laufend über die erste Stufe 3 in den Kreislauf eingespeist. Gleichzeitig werden die aus dem Synthesegas S erzeugten Produkte Q kontinuierlich aus der dritten Stufe abgezogen, sowie die verschiedenen Reststoffe H, K, J laufend aus dem Kreislauf entfernt.

Die erste Stufe umfasst im gezeigten Beispiel einen ersten Druckreaktor 30, in welchem unter Druck eine Pyrolyse des kohlenstoffhaltigen Ausgangsmaterials A stattfindet. Das Ausgangsmaterial A wird dazu über eine geeignete Druckschleuse 241 in den unter Druck stehenden Pyrolysereaktor 30 eingebracht. In der gezeigten Ausgestaltungsform besteht der Pyrolysereaktor 30 aus einem horizontalen Druckkörper 31, in welchem die horizontale Förderung des Materials entlang des Reaktors während der Pyrolyse durch einen schematisch dargestellten Vorschubrost 32 mit hin und her bewegten Rostplatten erfolgt. Jede andere für den kontinuierlichen Vorschub des zu verarbeitenden Ausgangmaterials geeignete Förderungsvorrichtung ist ebenfalls anwendbar, beispielsweise Walzenroste, Kettenförderer, Förderschnecken, etc., sind ebenfalls einsetzbar. Auch ein Drehrohrofen kann eingesetzt werden.

Als Energie lieferndes Ausgangsmaterial A kann eine Vielzahl kohlenstoffhaltiger Substanzen dienen, insbesondere Kohle, Biomasse, Abfälle sowie andere heterogene Substanzen wie beispielsweise verunreinigtes Erdreich. Auch schwierig zu verwertende fest-flüssige erdölhaltige Substanzen wie Ölschiefer, Ölsand, oder Ölschlamm können in einem erfindungsgemässen Verfahren verwertet werden. Als Zuschlagstoffe A' können auch gasförmige kohlenstoffhaltige Nebenprodukte der Erdölindustrie verwendet werden, die ansonsten nicht weiter verwertet werden können und abgefackelt werden müssten.

Der Brennwert der Ausgangsmaterialien, der Kohlenstoffgehalt, Wassergehalt, sowie der Gehalt an nicht brennbaren Reststoffen wie Metall, Glas und Keramik, kann stark variieren. Das Ausgangsmaterial kann auf eine für eine bestimmte Verwertungs-Anlage geeignete Stückgrösse verkleinert werden, wobei sich die bevorzugte Stückgrösse aus der Konsistenz des Materials und aus der konkreten Ausgestaltung des ersten Druckreaktors bzw. des reaktorinternen Fördersystems ergibt. Zur Verarbeitung mit einem Vorschubrost ist beispielsweise eine Stückgrösse von ca. 5-10 cm gut geeignet.

Im Pyrolysereaktor 30 wird das Material bei einer Temperatur von ca. 300-800°C und einem Druck von 10 bis 60 bar kontinuierlich durch den Druckreaktor 30 transportiert und dabei unter Sauerstoffausschluss pyrolysiert. Die Temperatur wird dabei unter anderem so gewählt, dass neben der Aufrechterhaltung der Pyrolysereaktion der gewünschte Betriebsdruck gehalten wird, zum einen aufgrund der Ausdehnung der Gase aufgrund der Temperatur, und zum anderen aufgrund der Neuproduktion von Pyrolysegasen. Eine Minimaltemperatur von 450 °C stellt eine stetige Abreaktion von freien Sauerstoffverbindungen während der Pyrolyse sicher. Besonders gut geeignet sind eine Betriebstemperatur von 500-600 °C und ein Betriebsdruck zwischen 10 und 25 bar. Die für die Pyrolysereaktionen notwendige thermische Energie stammt zum einen durch aus dem heissen Feedback-Gasstrom S2 aus dem zweiten Reaktor 40, auf den nachfolgend noch eingegangen wird, und die Zugabe von Prozessdampf B der Aufrechterhaltung der Betriebstemperatur des ersten Reaktors. Ebenfalls vorhanden sein kann eine externe Wärmezufuhr wie beispielsweise ein Wärmetauscher oder eine externe Heizung. Letztere ist auch vorteilhaft bei der Inbetriebnahme der Verwertungsanlage 2 aus dem Kaltzustand.

Rücklaufgas R aus der dritten Stufe (Synthese-Stufe) 5 wird nach dem Passieren eines Verdichters 212 dem ersten Druckreaktor 30 zugeführt. Das Rücklaufgas R enthält vor allem Kohlendioxid, sowie Wasserdampf und in der Synthese-Stufe nicht reagiertes Kohlenmonoxid und Wasserstoff. Um den Prozess steuern zu können, kann zusätzlicher Kohlenstoff mit hohem Heizwert in den Reaktor 30 eingebracht werden, beispielsweise in Form von Kohle oder Schweröl. Diese Zuschlagstoffe A' können bereits vorgängig dem Ausgangsmaterial A zugeschlagen werden, oder separat in den Reaktor 30 eingebracht werden. Die Vermengung von viskosen Substanzen A' mit festem Ausgangsmaterial A erleichtert die Förderung innerhalb des Reaktors. Flüssige Zuschlagstoffe A' erhöhen auch die Menge an Pyrolysegas, und damit den Betriebsdruck.

Bei der Pyrolyse der ersten Stufe 3 entsteht Pyrolysekoks D, der im Wesentlichen aus festem Kohlenstoff und anorganischen Reststoffen besteht, welcher am Ende des Druckreaktors 30 abgeführt wird. Die bei der Pyrolyse entstehenden Pyrolysegase E beinhalten sowohl gasförmige, als auch bei Raumtemperatur flüssige und feste Substanzen. Die Zusammensetzung der Pyrolysegase E hängt naturgemäss stark von den Ausgangsmaterialien ab, und enthält gegebenenfalls auch Schadstoffe.

Der Pyrolysekoks D wird unter Druck in den Druckreaktor 40 der zweiten Stufe 4 gefördert. Geeignet ist wiederum beispielsweise eine geschlossene Förderschnecke. Die Pyrolysegase E werden über eine separate Transportleitung ebenfalls in den zweiten Druckreaktor 40 gefördert. Ein in der Transportleitung angeordneter Verdichter 211 fördert die Pyrolysegase in den unter einem höheren Betriebsdruck stehenden zweiten Druckreaktor 40.

In der zweiten Stufe 4 liegt die Betriebstemperatur zwischen 600 und 1600 °C. In dieser zweiten Stufe erfolgt nun die Vergasung des festen Kohlenstoffs im Pyrolysekoks D mit Kohlendioxid und gegebenenfalls Sauerstoff und/oder Wasserdampf als Vergasungsmittel zu Kohlenmonoxid und Wasserstoff, gemäss den Reaktionen I, II und III.

Das Kohlendioxid stammt primär aus dem Rücklaufgas R. Es kann auch zusätzliches Kohlendioxid T in den Kreislauf eingespeist werden. Der Wasserdampf besteht primär aus der Restfeuchte des Ausgangsmaterials A. Es kann auch Prozessdampf B eingespeist werden.

Die für den Ablauf dieser endothermen Reaktionen notwendige thermische Energie stammt beispielsweise aus einer partiellen Oxidation des festen Kohlenstoffs (Reaktion III) mit in den zweiten Druckreaktor 40 geleitetem Sauerstoff F. Zum Aufstarten der erfindungsgemässen Anlage und zur Steuerung des Prozesses kann es notwendig sein, dem zweiten Reaktor 40 zusätzliche Brennstoffe G zuzuführen, wie beispielsweise Koks, Öl oder Erdgas, und/oder die Sauerstoffzufuhr, um damit die Wärmeproduktion vorübergehend zu erhöhen.

Das für die spätere Synthese in der dritten Stufe 5 wichtige Verhältnis zwischen Kohlenmonoxid und Wasserstoff ist durch die Wassergas-Shift-Reaktion IV gegeben, und kann durch Zugabe von Prozessdampf B auf die rechte Seite hin beeinflusst werden, Es ist jedoch vorteilhaft, die Gesamtmenge an Wasser im System möglichst gering zu halten, und stattdessen zusätzlichen Wasserstoff direkt in die dritte Stufe einzuleiten.

Im gezeigten Beispiel einer Verwertungsanlage 2 einer erfindungsgemässen Kraftwerksanlage 1 umfasst die zweite Stufe ebenfalls einen horizontalen Druckkörper 41, in welchem die Förderung des Pyrolysekoks innerhalb des Reaktors 40 durch einen Vorschubrost 42 erfolgt. Wiederum sind auch andere Fördersysteme möglich, wie sie auch bereits für den ersten Druckreaktor 30 besprochen worden sind. Dies hat den Vorteil, dass der Pyrolysekoks ohne weitere Vorbereitung in der zweiten Stufe prozessiert werden kann. Die reaktive Oberfläche des stückigen Pyrolysekoks ist im Vergleich einer ebenfalls möglichen Reaktion im Wirbelstrom vergleichsweise klein, was aber durch die aufgrund der hohen Massenkapazität des Druckreaktors vergleichsweise lange Verweildauer im Reaktor 40 ausgeglichen wird.

Grundsätzlich kann der zweite Reaktor auch anders ausgestaltet sein. Beispielsweise könnte der Pyrolysekoks vorangehend zerkleinert oder gemahlen werden, was dann eine Vergasung des Koks im Wirbelstrom oder Flugstrom ermöglichen würde. Aufgrund der grösseren Oberfläche erlauben solche Reaktoren schnellere Reaktionsgeschwindigkeiten, was jedoch gleichzeitig mit geringeren Massenkapazitäten einhergeht. Zudem erfordern solche Anlagen eine präzisere Steuerung der Gasstromgeschwindigkeit.

Der für die partielle Verbrennung notwendige Sauerstoff F und gegebenenfalls der Prozessdampf B wird in das durch den Pyrolysekoks gebildete Glutbett eingeblasen, wodurch die notwendige thermische Energie erzeugt und der Reaktor 40 auf Betriebstemperatur gehalten wird. Anstatt reinem Sauerstoff könnte auch Luft erwendet werden, wobei jedoch der Luftstickstoff den innerhalb der erfindungsgemässen Anlage kreisenden Gasmateriestrom aufbläht. Dies reduziert die Effizienz der Anlage wesentlich, so dass reiner Sauerstoff in jedem Fall vorzuziehen ist. Zudem verhindert die Abwesenheit von Stickstoff im System auch die Bildung von Stickoxiden.

Die Pyrolysegase E werden im in Figur 2 gezeigten Ausführungsbeispiel einer Verwertungs-Anlage 2 in die Gasphase oberhalb des Glutbetts im Druckreaktor 40 eingeblasen, wo die in den Pyrolysegasen E enthaltenen mehratomigen Moleküle bei den herrschenden hohen Temperaturen sehr schnell gecrackt und zerlegt werden. Das in der zweiten Stufe gebildete Synthesegas S enthält darum im Wesentlichen keine organischen Moleküle mehr, und kann für die Fischer-Tropsch-Synthese in der dritten Stufe verwendet werden. Auch Schadstoffe wie zum Beispiel Dioxin werden zerlegt. Die Sauerstoffzufuhr F ins Glutbett und der Eintrittsort der Pyrolysegase E in den Druckreaktor werden so gewählt, dass keine Bildung von Dioxinen erfolgen kann. Ebenso sollte im austretenden Synthesegas kein Sauerstoff vorhanden sein.

Für unproblematische Ausgangsstoffe, wie beispielsweise Holzschnitzel oder Stroh oder andere unbelastete Biomassen ist es auch möglich, die Pyrolysegase E vorgängig mit Sauerstoff in einem separaten Brenner zu verbrennen, und die heissen Abgase zwecks Zufuhr thermischer Energie ebenfalls ins Glutbett zu leiten, oder unverbrannt direkt ins Glutbett einzublasen, wo sie ebenfalls oxidiert werden.

Am Ende des Druckreaktors 40 verbleiben Reststoffe in Form von Asche und inerten Reststoffen sowie gegebenenfalls nicht prozessiertem Kohlenstoff. Vorzugsweise sind die Temperaturen so gewählt, dass die Asche verschlackt, so dass die resultierende Schlacke H später unproblematisch deponiert werden kann. Es können auch Zuschlagstoffe beigegeben werden, welche den Ascheschmelzpunkt erniedrigen. Zu diesem Zweck kann beispielsweise dem Ausgangsmaterial A Kalkpulver zugeschlagen werden. Die Schlacke wird aus dem zweiten Druckreaktor 40 über eine geeignete Druckschleuse 242 aus dem Druckbereich der Verwertungsanlage 2 abgeführt.

Der Synthesegas-Strom S wird aus dem zweiten Druckreaktor 40 abgeführt. Ein Hauptteil S1 wird einem Zyklon 22 zugeführt, wo Staub K, hauptsächlich bestehend aus Restkoks und Asche, abgetrennt wird. Der Reststaub K kann in den ersten 30 oder zweiten 40 Druckreaktor zurückgeführt werden, oder er wird aufbereitet und/oder entsorgt. Anstelle eines Zyklons kann auch eine andere geeignete Gasstromreinigungsvorrichtung verwendet werden.

Anschliessend an die Entfernung des Reststaubs K wird der Synthesgas-Strom durch einen geeigneten Wärmetauscher 231 geleitet, wo der Gasstrom unter Erzeugung von Prozessdampf C2, B auf eine Temperatur abgekühlt wird, die für die Fischer-Tropsch-Synthese in der dritten Stufe geeignet ist. Aufgrund der tieferen Temperaturen sinkt der Druck und verschiebt sich das Gleichgewicht der Reaktionen I, II und IV, wodurch der Anteil an Kohlendioxid im Synthesegas wieder steigt. Ebenfalls kann fester Kohlenstoff L in Form von Graphit aus dem Gasstrom abscheiden. Der Kohlenstoff L kann als Ausgangsmaterial A, A' zurück in den Kreislauf geführt werden, oder als Wertstoff anderweitig verwertet werden. Falls der Kohlenstoff L nicht ausgeschieden wird, gelangt er mit dem Synthesegasstrom in dem Fischer-Tropsch-Reaktor 50, wo er zusammen mit dem in der Fischer-Tropsch-Reaktion als Nebenprodukt entstehenden Kohlenstoff ausgeschieden bzw. abfiltriert werden kann. Je nach Ausgangsmaterial kann eine Gastromaufbereitung vorgesehen sein, um störende Stoffe im Synthesegas zu entfernen.

Das Synthesegas S wird nun über eine Druckregulierung 25 einem dritten Druckreaktor 50 der dritten Stufe 5 zugeführt, in welcher eine Fischer-Tropsch-Synthese durchgeführt wird. Die Druckregulierung 25 reduziert den Druck auf den für die dritte Stufe gewünschten Wert. Zur Einstellung des gewünschten Verhältnisses Kohlenmonoxid/Wasserstoff kann zusätzlicher Wasserstoff N in den Fischer-Tropsch-Reaktor 50 geleitet werden. Ebenfalls werden die notwendigen Festkörper-Katalysatoren P zugeführt.

In der Fischer-Tropsch-Synthese der dritten Stufe reagieren das Kohlenmonoxid und der Wasserstoff stark exotherm (ca. 158 kJ/mol pro Kohlenwasserstoffkettenglied bei 250 °C) an heterogenen Katalysatoren (beispielsweise Eisen-, Kobalt-, Ruthenium-, Nickelkatalysatoren) zu Alkanen, Olefinen, Alkoholen, Aldehyden und anderen Kohlenwasserstoffverbindungen und Derivaten. Nebenprodukte sind Methan und fester Kohlenstoff, die ebenfalls in stark exothermen Reaktionen entstehen. Die genauen Parameter der Fischer-Tropsch-Synthese, insbesondere Druck und Temperatur, hängen primär von den herzustellenden Produkten ab, und sind für das grundlegende Funktionsprinzip einer erfindungsgemässen Anlage bzw. das erfindungsgemässe Verfahren nicht direkt relevant. Tendenziell führen höhere Prozesstemperaturen zu kürzeren Kettenlängen und vermehrter Kohlenstoffabscheidung, während höhere Drücke zu längere Kettenlängen führen. Daneben haben die vor allem vorliegenden Partialdrücke von Kohlenmonoxid, Wasserstoff und Wasser einen starken Einfluss auf die Syntheseprodukte.

Für die Synthesestufe geeignet sind beispielsweise Tieftemperatur-Fischer-Tropsch-Verfahren, die bei beispielsweise bei 210 bis 250 °C betrieben werden, und vor allem dieselähnliche Produkte und langkettige Anteile in Form von Wachsen liefern. Letztere können dann zum Beispiel durch Hydrocracking weiter verwertet werden. Hochtemperaturverfahren mit Temperaturen zwischen 320 bis 350 °C wiederum liefern erhebliche Anteile an Methan, kurzkettigen Alkanen und Alkenen, sowie höhere Anteile an Leichtbenzin.

Für Tieftemperaturverfahren eignen sich beispielsweise Rohrbündelreaktoren, in welchen das Synthesegas durch mit Katalysator befüllte, gekühlte Rohre von oben nach unten strömt. Rücklaufgas und Produkte verlassen das Rohr bodenseitig. Besonders geeignet sind moderne Suspensationsreaktoren (schematisch dargestellt in Figur 2), in welchen der feste Katalysator fein verteilt im flüssigen Produkt schwimmt (Slurry). Flüssige Reaktionsprodukte werden aus der flüssigen Phase abfiltriert, während gasförmige Produkte den Reaktor als Teil des Rücklaufgases R verlassen. Die Wärmeabfuhr erfolgt über eingehängte Kühlrohre 51, unter Erzeugung von Prozessdampf C1, B. Suspensationsreaktoren weisen eine einfachere Bauweise auf als Rohrbündelreaktoren, und sind darum kostengünstiger. Der Katalysator kann effizienter genutzt werden, und ist während des laufenden Betriebs austauschbar, was beim erfindungsgemässen zyklischen Verfahren vorteilhaft ist.

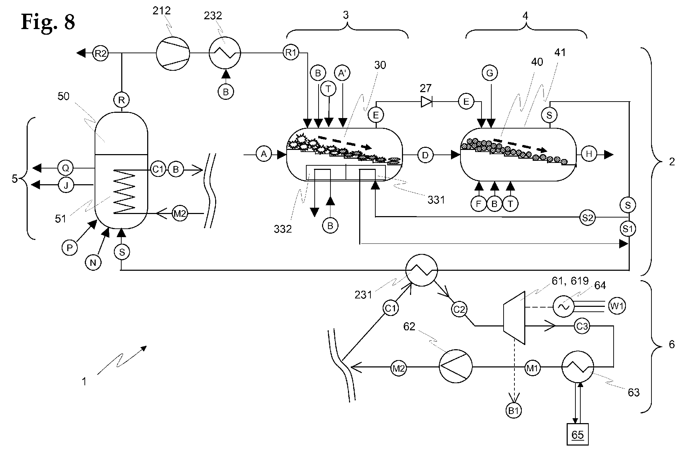

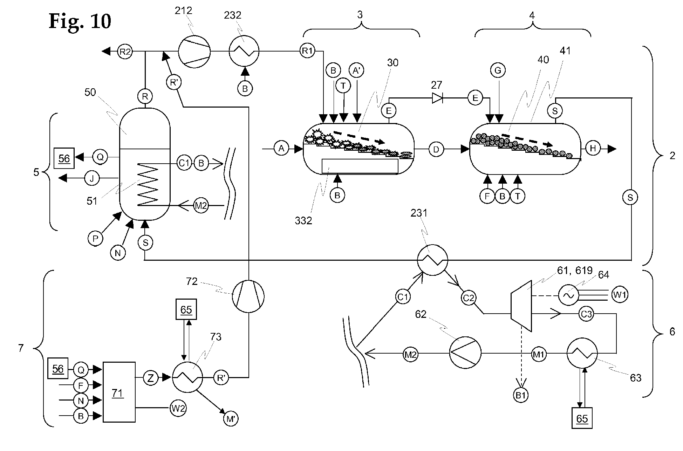

Der durch die Kühlvorrichtung 51 gewonnene Prozessdampf C1, B beinhaltet eine erhebliche thermische Energie, ist jedoch noch nicht heiss genug für eine effiziente Verwertung in einer Dampfturbine. Er wird deshalb vorteilhaft für die Prozessdampfgewinnung C2 zum Beispiel im Wärmetauscher 231 verwendet, um die allgemeine Energieeffizient der Anlage zu erhöhen und damit die Kohlendioxid-Bilanz weiter zu verbessern. Auf das entsprechende Zusammenspiel zwischen Verwertungsanlage 2 und dem zweiten Anlagenteil 6 wird in den Figuren 8 und 10 genauer eingegangen.

Der Rücklaufgasstrom R, welcher den Fischer-Tropsch-Reaktor 50 verlässt, enthält neben unreagiertem Kohlenmonoxid und Wasserstoffgas noch Wasserdampf, Kohlendioxid, und gasförmige Reaktionsprodukte. Ein Anteil an leichtflüchtigen Kohlenwasserstoffen kann daraus beispielsweise mit Hilfe einer Kühlkolonne (nicht dargestellt) auskondensiert werden. Ebenso kann Wasser auskondensiert und aus dem Rücklaufgas entfernt werden. Ein Teil R3 des Rücklaufgases kann auch in den dritten Reaktor zurückgeleitet werden, wodurch verbliebene Synthesegasanteile nachreagiert und die Ausbeute erhöht werden kann. Aus dem verbliebenen Rücklaufgasstrom kann ein Teil R2 als Verfahrensprodukt abgetrennt werden. Der verbleibende Rücklaufgasstrom R1 wird in einem Verdichter 212 verdichtet und wieder zurück in den ersten Reaktor 30 geführt.

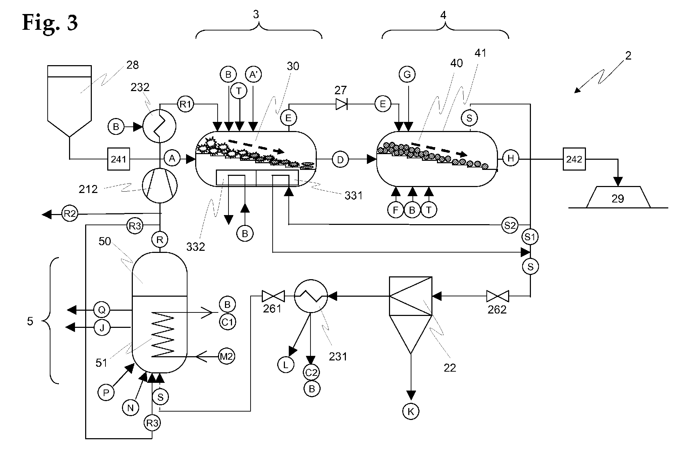

Die zyklische Förderung des Gasstroms innerhalb der Verwertungsanlage 2 erfolgt vor allem aufgrund der herrschenden Druckdifferentiale entlang des Kreislaufs. Diese werden primär durch die zwei Verdichter 211, 212 erzeugt. Je nach Auslegung der Anlage kann auf einen der beiden Verdichter verzichtet werden, was die Gesamtkosten der Anlage senkt. Beinhaltet die Anlage nur einen Verdichter (wie beispielsweise im nachfolgend besprochenen zweiten Ausführungsbeispiel einer Verwertungsanlage in Figur 3), so hat die Anordnung vor dem ersten Reaktor 30 den Vorteil, dass der entsprechende Verdichter 212 ein geringeres Gasvolumen verdichten muss, als ein Verdichter 211 zwischen der ersten und zweiten Stufe, wo zusätzlich die Pyrolysegase hinzukommen und das Gesamtvolumen aufgrund der höheren Temperatur grösser ist, oder gar zwischen der zweiten und dritten Stufe.

Wird auf den Verdichter 211 verzichtet, so besteht zwischen den beiden Reaktoren 30, 40 nur ein geringes Druckgefälle, so dass die erste und zweite Stufe im wesentlichen bei gleichem Druck ablaufen. Der Gasstrom läuft dann also vom Verdichter 212 über den ersten 30, zweiten 40 und dritten Reaktor 50 zurück zum Verdichter 212. Wird hingegen auf den Verdichter 212 verzichtet, ist der Druck innerhalb des dritten und ersten Reaktors 50, 30 im Wesentlichen gleich. Es kann auch ein Verdichter zwischen der zweiten und der dritten Stufe angeordnet werden. Aus Gründen der Entropie muss mindestens ein Verdichter oder sonst ein Fördermittel vorhanden sein, um den Gasstrom zu fördern und das Verfahren am Laufen zu halten. Zum Ausgleich von vorübergehenden Schwankungen in der Gasproduktion aufgrund von heterogenem Ausgangsmaterial können entlang des Gaskreislaufs E, S, R Druckspeicher vorgesehen sein.

Falls die Verwertungs-Anlage 2 aus Figur 2 klein ausgeführt wird, und entsprechend der Volumenstrom E zwischen dem ersten 30 und dem zweiten 40 Druckreaktor vergleichsweise gering ist, kann der Verdichter 211 mit vertretbarem Energieaufwand einen Druckunterschied von mehreren Bar erzeugen. Die erste Stufe könnte dann bei einem wesentlich niedrigeren Druck als die zweite Stufe gefahren werden. Die erste Stufe kann sogar bei Normaldruck oder sogar Unterdruck durchgeführt werden.

Zur Inbetriebnahme der Verwertungsanlage 2 wird der Kreislauf und die drei Stufen mit einem sauerstofffreien Gas, vorzugsweise Kohlendioxid, gespült und befüllt. Anschliessend wird der vorgängig mit Koks befühlte zweite Druckreaktor 40 aufgeheizt, beispielsweise mit Gasbrennern. Der zweite Reaktor wird dazu vom Kreislauf abgetrennt, durch Schliessen der entsprechenden Verbindungen. Während des Aufheizens auf die gewünschte Betriebstemperatur wird die Förderung 42 des Koks innerhalb des Druckreaktors 40 noch nicht eingeschaltet. Gegebenenfalls kann im Kreislauf ein temporärer Kurzschluss zwischen Wärmetauscher 231 und Druckreaktor 40 vorgesehen sein (nicht dargestellt), um das erwärmte Gas im System zirkulieren und den gesamten Anlagenabschnitt gleichmässig aufwärmen zu können. Der Druck wird ebenfalls auf den Sollwert erhöht.

Parallel dazu wird der ebenfalls vorgängig mit Koks befüllte erste Druckreaktor 30 vom Kreislauf abgetrennt, und auf die vorgesehene Betriebstemperatur der ersten Stufe aufgeheizt. Der Druck wird ebenfalls auf den gewünschten Wert für die erste Stufe gebracht. Die Materialförderung 32 im ersten Reaktor bleibt noch ausgeschaltet. Das Aufheizen sollte jedoch vorzugsweise ohne Ausgangsmaterial erfolgen, da eine Pyrolyse des Ausgangsmaterials unterhalb einer minimalen sicheren Betriebstemperatur von 450 °C zur Bildung explosiver Gemische führen kann. Der Koks hingegen ist bereits pyrolysiert, und dient lediglich der Zufuhr von Koks in die zweite Stufe, wenn der Kreislauf später in Gang gesetzt wird.

Der Fischer-Tropsch-Reaktor 50 wird ebenfalls vom Kreislauf abgetrennt auf die Betriebsbedingungen hochgefahren. Nach Erreichen der Betriebsbedingungen in den verschiedenen Stufen der Verwertungsanlage werden die verschiedenen Fördersysteme 32, 42 langsam hochgefahren, der Kreislauf geöffnet, und die Verdichter 211, 212 in Betrieb gesetzt, so dass schlussendlich ein Gleichgewichtszustand der Verwertungsanlage 2 bei den gewünschten Betriebsparametern resultiert.

Eine weitere Ausführungsform einer Verwertungsanlage 2 einer erfindungsgemässen Kraftwerksanlage ist in Figur 3 dargestellt. Im Gegensatz zu Figur 2 ist zwischen dem ersten Druckreaktor 30 und dem zweiten Druckreaktor 40 kein Verdichter angeordnet, sondern lediglich ein Rückschlagventil 27, auf das jedoch gegebenenfalls auch verzichtet werden kann. Der Gasstrom wird durch das durch den Verdichter 212 erzeugte Druckgefälle durch die Anlage gefördert. Da diese vorteilhafte Variante nur mit einem einzigen Verdichter 212 auskommt, der zudem ein geringeres Durchsatzvolumen aufweisen muss, sind die Gesamtkosten der Anlage 2 geringer.

In der gezeigten Variante wird der abgezweigte Synthesegas-Strom S2 nicht direkt zurück in den ersten Reaktor 30 geleitet, sondern stattdessen durch eine Heizvorrichtung 331 des Druckreaktors 30 geführt und anschliessend wieder mit dem Synthesegas S1 vereint. Alternativ oder zusätzlich kann eine weitere Heizvorrichtung 332 vorgesehen sein, welche mit überhitztem Prozessdampf B betrieben wird.

Ein Wärmetauscher 232 ist im Rücklaufgasstrom R1 angeordnet, und dient der Erhitzung des Rücklaufgasstromes R1 durch Prozessdampf B. Der Rücklaufgasstrom dient in dieser Ausführungsform damit ebenfalls der Wärmezufuhr in den ersten Druckreaktor 30.

Im gezeigten Beispiel ist vor dem dritten Druckreaktor 50 keine Druckreduzierung vorgesehen. Die Drucksteuerung in der dritten Stufe erfolgt dabei direkt über die Drucksteuerung in der zweiten Stufe und den nachfolgenden Druckabfall aufgrund der Abkühlung des Synthesegasstroms S im Wärmetauscher 231, sowie den Verdichter 212.

Das in den Figuren 2 und 3 dargestellte erfindungsgemässe Verfahren beruht auf einem zyklischen Stofffluss durch die drei Stufen 3, 4, 5 der Verwertungsanlage 2, wobei kohlenstoffhaltiges Ausgangsmaterial A als Kohlenstofflieferant und Energieträger in den Kreislauf eingespeist und die Produkte Q der Synthese-Stufe als Betriebsstoff für den dritten Anlagenteil 7 der erfindungsgemässen Kraftswerksanlage 1 abgezweigt werden. Die Schlacke H als Restmaterial, sowie Wasserdampf im Rücklaufgas R2 werden laufend aus dem Kreislauf entfernt. Der in den Wärmetauschern entstehende Prozessdampf B, C1, C2 wird zur Energieproduktion im zweiten Anlagenteil 6 verwendet. Daneben wird der Prozessdampf auch für den Betrieb der Verwertungsanlage 2 verwendet, und erhöht so deren Effizienz und Effektivität.

Im Wesentlichen wird in einem erfindungsgemässen Verfahren bzw. einer erfindungsgemässen Kraftswerksanlage 1 aus einem energiereichen aber heterogenen und schwer verwertbaren fest Ausgangsmaterial A überhitzter Dampf zum Dauerbetrieb einer ersten Generatorstufe 6 erzeugt, sowie Betriebsstoff Q zum flexiblen Betrieb einer zweiten Generatorstufe 7. Die für den Betrieb der Verwertungsanlage 2 notwendige Energie stammt aus der partiellen Verbrennungsreaktion in der zweiten Stufe, wobei ein Überschuss der dort erzeugten chemischen Energie (in Form des Synthesegases) später in der exothermen Fischer-Tropsch-Reaktion der dritten Stufe wieder in thermische Energie in Form von Prozessdampf C3 umgewandelt wird.

Aufgrund des kreisenden Materiestroms im Verfahren liegt während des Betriebs der Anlage 2 ein dynamisches Gleichgewicht vor. Die notwendigen Werte der verschiedenen Parameter (Druck, Temperatur, chemische Zusammensetzungen, Fördergeschwindigkeiten etc.) in den einzelnen Teilen der Anlage werden unter anderem durch die Art des verwendeten Ausgangsmaterials bestimmt. Um trotz heterogenem Ausgangsmaterial einen konstanten Betriebszustand zu halten, können diverse Betriebsparameter gesteuert werden.

Für die Produktion der Kohlenwasserstoffe und anderen Produkte in der dritten Fischer-Tropsch-Stufe 5 sind der Druck und die Temperatur im dritten Reaktor 50 die ausschlaggebenden Parameter. Der Druck kann kurzfristig mit dem Verdichter 212 kontrolliert werden, indem dessen Leistung erhöht oder erniedrigt wird. Die Temperatur kann wiederum durch die Kühlleistung des Wärmetauschers 51 gesteuert werden. Langfristig kann der Druck über den Druck im Synthesegasstrom S gesteuert werden, indem zum einen der Betriebsdruck und die Temperatur in der zweiten Stufe geändert wird, und zum anderen die Kühlleistung des Wärmetauschers 231 gesteuert wird, und damit der Temperatur- und Druckabfall im Synthesegasstrom S.

Die Steuerung einer Verwertungs-Anlage 2 ist vergleichsweise einfach, da die Anlage in einem Gleichgewicht mit Feedback fährt, und zur Steuerung einiger weniger relevanter Parameter eine Mehrzahl von Parametern, die einzelnen Betriebsparameter der verschiedenen Anlagenbestandteile verändert werden können, welche das Gleichgewicht langsam oder schnell beeinflussen können.

Das Verfahren wird vorzugsweise mit einem erhöhten Kohlendioxidanteil durchgeführt. Dies verschiebt unter anderem das Reaktionsgleichgewicht IV nach links (mehr Kohlenmonoxid). Ermöglicht wird ein solcher erhöhter Kohlendioxidgehalt bei gleichzeitig trotzdem möglichst hohen absoluten Mengen an Kohlenmonoxid und damit an Verarbeitungsleistung durch den erhöhten Betriebsdruck der erfindungsgemässen Anlage zwischen 10 und 60 bar. Höhere oder tiefer Drücke sind ebenfalls möglich, aber weniger effizient.

Die erfindungsgemässe Kraftwerksanlage 1 kann in Bezug auf verschiedene Aspekte optimiert werden. So kann beispielsweise die Betriebstemperatur der zweiten Stufe 3 der Verwertungsanlage 2 gesenkt werden, um den Mengen-Durchsatz im zweiten Reaktor 40 zu erhöhen. Dies führt dann gegebenenfalls dazu, dass gewisse flüchtige Stoffe im Pyrolysegas E nicht mehr gecrackt werden und mit dem Synthesegas S in den Fischer-Tropsch-Reaktor 50 gelangen. So kann beispielsweise Benzol aus dem Ausgangsmaterial, beispielsweise aus Schweröl, in kleineren Mengen in die Produkte der Fischer-Tropsch-Synthese gelangen. Dort verbleiben diese Substanzen als Teil eines flüssigen Betriebsstoffes Q, können wenn nötig jedoch auch abgetrennt werden.

In einer weiteren möglichen Variante des erfindungsgemässen Verfahrens wird der Tieftemperatur-Fischer-Tropsch-Reaktor der dritten Stufe durch einen Hochtemperatur-Fischer-Tropsch-Reaktor ersetzt, in welchem der Katalysator als verwirbelter Flugstaub vorhanden ist. Die bei der Hochtemperatur-Fischer-Tropsch-Synthese bevorzugt gebildeten gasförmigen, kurzkettigen Kohlenwasserstoffe, die nach einer ersten Kondensationsstufe im Rücklaufgas verbleiben, werden von den kleineren Molekülen des Rücklaufgases wie Kohlendioxid, Kohlenmonoxid, Wasserstoff durch Permeationsgasfilter abgetrennt. Solche Systeme sind beispielsweise aus der petrochemischen Industrie zur Reinigung von Erdgas bekannt. Im vorliegenden Fall dienen sie dazu, eine erste, kohlenwasserstoffreiche Gasphase und eine zweite, kohlenwasserstoffarme Gasphase zur erzeugen. Die kohlenwasserstoffreiche Gasphase wird weiter als Betriebsstoff für die zweite Generatorstufe verwertet. Die kohlenwasserstoffarme und kohlendioxidreiche zweite Gasphase wird als Rücklaufgas zurück in den Kreislauf geschickt.

In einer anderen Variante einer erfindungsgemässen Anlage beinhaltet die dritte Stufe 5 anstatt eines Fischer-Tropsch-Reaktors einen Liquid-Phase-Methanol-Synthese-Reaktor. Die aus dem Stand der Technik bekannte Liquid-Phase-Methanol-Synthese ist besonders dazu geeignet, Methanol in hoher Ausbeute aus Synthesegas mit höherem Anteil an Kohlendioxid herzustellen. Die Synthese findet in einem "Slurry-Bubble-Column-Reactor", in welchem das Synthesegas in eine Aufschlämmung des pulverförmigen Katalysators in einem inerten Mineralöl eingeblasen wird. Die Reaktion ist stark exotherm, so dass eine Kühlvorrichtung notwendig ist. Das erzeugte gasförmige Methanol verlässt den Druckreaktor zusammen mit unreagiertem Synthesegas. Nach dem Abscheiden von mitgeschlepptem Mineralöl und Katalysator wird das Methanol auskondensiert und als Betriebsstoff Q für die zweite Generatorstufe verwendet.

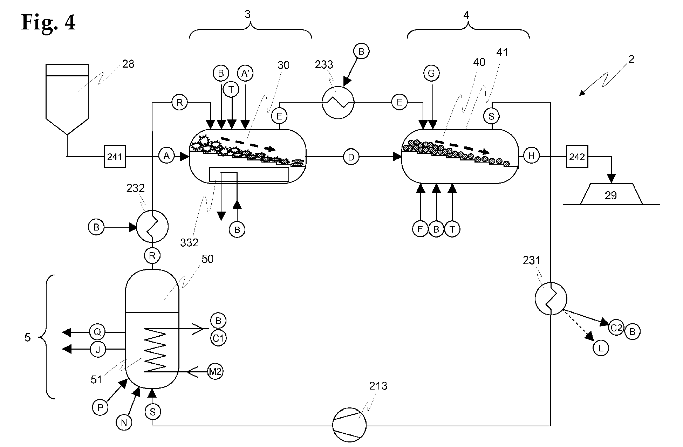

Figur 4 zeigt schematisch noch eine vorteilhafte Ausführungsform einer Verwertungsanlage 2 einer erfindungsgemässen Kraftwerksanlage. Zwischen der ersten Stufe 3 und der zweiten Stufe 4 ist ein Wärmetauscher 233 angeordnet, welcher dazu dient, die Pyrolysegase E vor dem Eintritt in den zweiten Druckreaktor 40 mit Prozessdampf auf die Betriebstemperatur der zweiten Stufe zu erhitzen. Der Verdichter 213 ist in der Transportleitung des Synthesegases S angeordnet, nach einem Wärmetauscher 231. Obwohl der Massestrom an dieser Stelle der Anlage am grössten ist, ist aufgrund der stark abgesenkten Temperatur nach dem Wärmetauscher 231 das für den Verdichter 213 zu bewältigende Gasvolumen kleiner, und die Betriebtemperatur für den Verdichter günstig.

In der dargestellten Verwertungsanlage 2 ist kein Zyklon zur Abtrennung von festen Bestandteilen K im Synthesegasstrom vorgesehen. Der Reststaub K tritt ungehindert in die dritte Stufe ein, wo er in der flüssigen Phase im Synthese-Reaktor 50 gebunden wird. Da der Reststaub in Kohlenwasserstoffen unlöslich ist, kann er ohne grossen Aufwand als Reststoff J ausfiltriert werden. Der Verzicht auf den Zyklonabscheider senkt die Kosten der Verwertungsanlage 2.

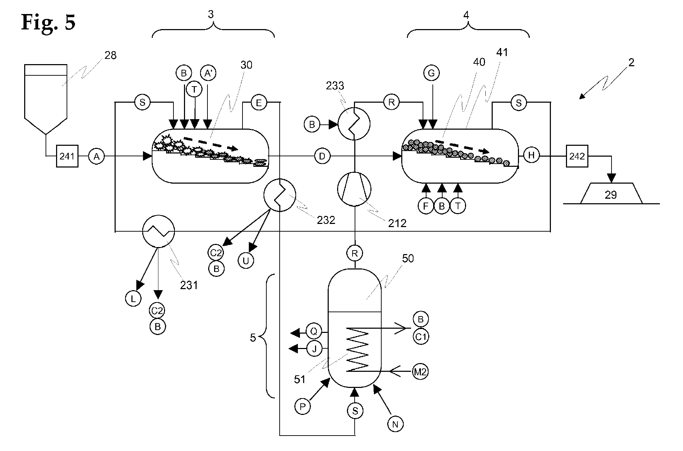

Eine weitere vorteilhafte Ausführungsform einer Verwertungsanlage 2 einer erfindungsgemässen Kraftwerksanlage 1 ist in Figur 5 dargestellt, welche besonders für die Herstellung von flüssigen Betriebstoffen Q aus unbelasteter Biomasse wie beispielsweise Holzschnitzeln geeignet ist. In dieser Variante werden die Pyrolysegase E nicht in die zweite Stufe 4 sondern in die dritte Stufe 5 geleitet, das Synthesegas S nicht in die dritte Stufe 5 sondern in die erste Stufe 3, und das Rücklaufgas R nicht in die erste Stufe 3 sondern in die zweite Stufe 4.

In der ersten Stufe 3 erhitzt der heisse Synthesegasstrom S das Pyrolysegut und hält die Betriebstemperatur aufrecht. Der aus der ersten Stufe austretende Pyrolysegasstrom E enthält dann neben den eigentlichen Pyrolysegasen auch den Synthesegasanteil aus der zweiten Stufe, welcher hier also eine Schleife über die erste Stufe macht.

In der zweiten Stufe 4 wird der Synthesegasanteil in den Pyrolysegasen E reagiert, während diejenigen Pyrolysegasanteile, welche nicht bereits im Wärmetauscher 232 auskondensiert U sind, sich in der flüssigen Phase des Synthese-Reaktors 50 lösen. Da bei einer direkten Verwendung der Produkte Q der dritten Stufe als Betriebsstoff für die zweite Antriebsvorrichtung 71 die Reinheitsanforderungen nicht sehr gross sind sind, kann so auf ein Cracken der Pyrolysegase verzichtet werden. Der Betriebstoff Q wird anschliessend nachgereinigt, um ungeeignete Reststoffe zu entfernen.

Der Rücklaufgasstrom R wird anschliessend verdichtet 212, erhitzt 233 und in die zweite Stufe 4 eingeleitet, so dass wieder ein Kreislauf gebildet wird. Da ein Cracken der in den Druckreaktor 40 eingeleiteten Gase nicht notwendig ist, kann die zweite Stufe bei geringerer Betriebstemperatur gefahren werden.

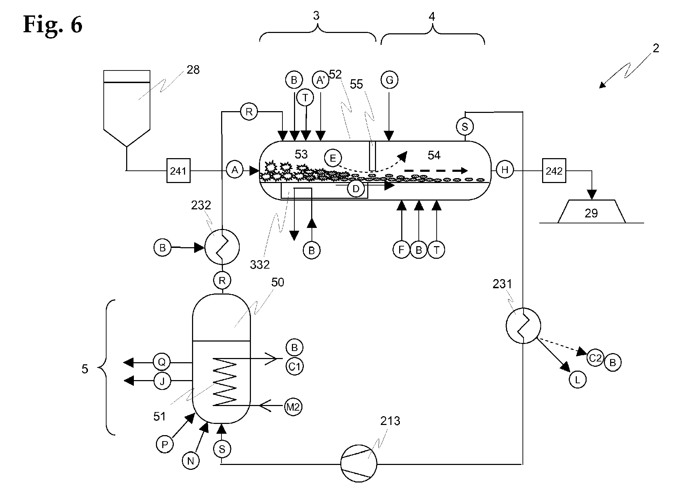

Figur 6 zeigt eine Ausführungsform einer erfindungsgemässen Anlage, bei der die erste und die zweite Stufe 3, 4 in einem gemeinsamen Druckreaktor 52 durchgeführt werden. Die Pyrolyse findet in einer ersten Kammer 53 des Reaktors 52 statt, die Vergasung in einer zweiten Kammer 54. Die beiden Kammern 53, 54 werden durch eine in Druckreaktor 52 angeordnete Trennwand 55 gebildet, mit einem Durchlass, durch welchen ein gemeinsames Fördersystem den Pyrolysekoks D fördert und das Pyrolysegas E strömt. Die Trennwand 55 dient vor allem dazu, die beiden Kammern 55, 54 thermisch zu isolieren, so dass in den beiden Stufen verschiedene Betriebstemperaturen gefahren werden können.

Eine mögliche Ausführungsform einer erfindungsgemässen Kraftwerksanlage 1 ist in Figur 8 schematisch dargestellt. Die gezeigte Anlage 1 umfasst eine einen ersten Anlagenteil 2 zur Verwertung des kohlenstoffhaltigen Ausgangsmaterials A, und einen zweiten Anlagenteil 6 zur Erzeugung einer im wesentlichen konstanten Grundmenge W1 an elektrischer und/oder mechanischer Energie.

Der Aufbau der Verwertungsanlage 2 entspricht im Wesentlichen der Ausführungsform aus Figur 3. Der Grundlast-Anlagenteil 6 umfasst eine Antriebsvorrichtung 61 in Form einer Dampfturbine 619 oder eine andere mit überhitztem Dampf C2 betreibbare Wärmekraftmaschine zur Erzeugung mechanischer Energie, und im gezeigten Beispiel einen durch diese angetriebene Generatorvorrichtung 64.

Im Wärmetauscher/Überhitzer 231, in welchem gleichzeitig das heisse Synthesegas S aus der zweiten Stufe 4 auf die Temperatur für die dritte Synthese-Stufe 5 heruntergekühlt wird, wird aus kälterem Dampf C1 überhitzter Dampf C2 erzeugt (ca. 550-600°C/50 bar). Nötigenfalls kann ein nachfolgender weiterer Wärmetauscher den Synthesegasstrom weiter abkühlen. Aus dem überhitzten Dampf C2 wird mit einer Dampfturbine mechanische Energie W1 erzeugt. Nach dem Entspannen in der Dampfturbine 619 wird der Abdampf C3 im Kondensator/Economizer 63 kondensiert M1, wobei die Abwärme über einen geeignet ausgestalteten Kühlkreislauf 65 abgeführt wird. Das entstehende Kondensat M1 ist vorzugsweise 60-70°C heiss, so dass zum einen das Wasser in der nachfolgenden Boilerstufe 51 nicht zu stark erhitzt werden muss. Gleichzeitig sollte das Wasser M1 nicht zu heiss sein, um Kavitation in der Pumpe 62 zu vermeiden. Das Kondensat M1 wird mit einer Pumpe 62 aus einem Zwischenspeicher (nicht dargestellt) in den Wärmetauscher/Boiler 231 gefördert, wo es wiederum zu Dampf C1 (ca. 250-300 °C/20 bar) verdampft wird, unter gleichzeitiger Kühlung der Synthesestufe 5. Der Dampf C1 wird in einem Dampfdom (nicht dargestellt) gespeichert, um zum einen vor dem Eintritt in den Überhitzter 231 verbliebenes Wasser abzutrennen, und zum zweiten einen Speicher zu bilden, aus dem Prozessdampf B für die verschiedenen Zwecke in der Verwertungsanlage 2 entnommen werden kann. Verluste im Kreislauf und Verbrauch von Prozessdampf B werden über eine Neuzufuhr von Wasser in den Kondensatspeicher ausgeglichen.

In einer alternativen Variante kann in der Dampfturbine 231 nach der Hochdruckstufe ein Teil des Dampfs als Prozessdampf B1 entnommen werden, was in Figur 8 als gestrichelter Pfeil dargestellt ist. Auf diese Weise kann eine grössere Menge an Dampf C2 zur Energiegewinnung genutzt werden, und erst danach der notwendige Prozessdampf bereitgestellt werden.

Der Abdampf von Prozessdampfverbrauchern wie beispielsweise den Wärmetauschern 232, 332 kann ebenfalls kondensiert und in das Speisewasser M1 zurückführt werden, so dass ein möglichst geschlossener Kreislauf resultiert.

Entsprechend lassen sich in einer erfindungsgemässen Anlage 1 die Wasserdampf-Kreisläufe auch anders durch die verschiedenen Wärmetauscher führen, um eine möglichst hohe Effizienz der Anlage 1 zu erreichen.

In einer erfindungsgemässen Kraftwerksanlage 1 nur mit einem Grundlast-Anlagenteil 6, wie sie beispielsweise in Figur 8 offenbart ist, können die in der Synthesestufe 5 entstehenden Produkte Q als Betriebsstoff für eine herkömmliche, mit fossilen Brennstoffen betreibbare Kraftwerksanlage 7 verwendet werden, beispielsweise Dieselgeneratoren oder Gasturbinengeneratoren, die zur Deckung von Spitzenlasten verwendet werden können. Die chemischen Betriebsstoffe Q dienen in einem solchen Fall dazu, losgelöst vom in einem Gleichgewichtzustand gefahrenen Grundsystem 2, 6 kurzzeitig sehr hohe Produktionsleistungen zu erreichen. So kann innerhalb eines sehr kurzen Zeitraumes die Gesamtleistung einer erfindungsgemässen Kraftwerksanlage 1 von beispielsweise 100% konstanter Grundlastproduktion c" auf beispielsweise 600% Spitzenlastproduktion e" erhöht werden.

Alternativ können die Produkte Q auch anderweitig verwendet werden, beispielsweise zur Herstellung von Treibstoffen oder als Edukte für die chemische Industrie.

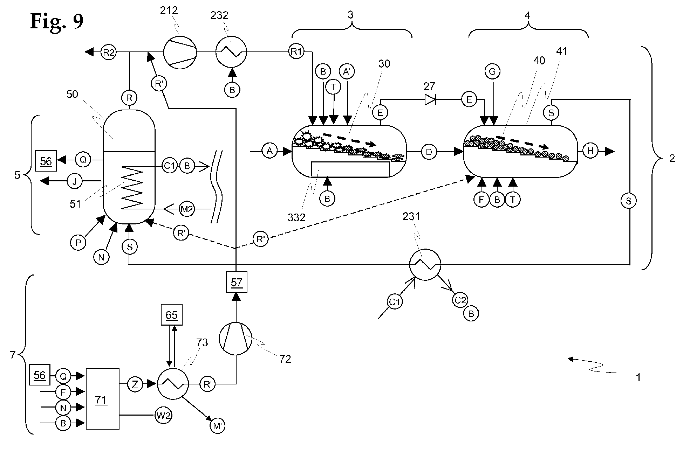

In einer besonders vorteilhaften Ausführungsform einer erfindungsgemässen Kraftwerksanlage 1, wie sie in schematisch in Figur 9 offenbart wird, ist ein dritter Anlageteil 7 zur Deckung von Spitzenlasten W2 derart ausgestaltet, dass das beim Betrieb des genannten Spitzenlast-Anlageteils 7 anfallende Kohlendioxid zurück in den Kreislauf der Verwertungsanklage 2 geführt wird, so dass keine Emissionen entstehen.

Eine Antriebsvorrichtung 71 erzeugt elektrische und/oder mechanische Energie W2 mittels chemischer Energieträger Q aus der Synthesestufe 5 der Verwertungsanlage 2. Die genannte Antriebsvorrichtung 71 kann beispielsweise eine Wärmekraftmaschine sein, in welcher die bei einer Oxidation der Betriebstoffe Q zu Kohlendioxid anfallende Wärme in mechanische Arbeit umgewandelt, zum Beispiel zum Betrieb einer Generatoranlage (nicht dargestellt), oder eine Brennstoffzellenanlage, in welcher die Oxidationsreaktion direkt zur Stromerzeugung W2 genutzt werden kann.

Die Betriebsstoffe Q werden vorteilhaft aus einem Zwischenspeicher 56 bezogen, beispielsweise einer Tankanlage oder einem Druckspeicher, um Bedarfsspitzen zu überbrücken. Eine solche Antriebsvorrichtung 71 weist einen geschlossenen Kreislauf auf, das heisst, sie verursacht keine Emissionen in die Atmosphäre. Die bei der Leistung der mechanischen Arbeit anfallenden Produktgase Z, die im Wesentlichen nur Kohlendioxid und gegebenenfalls noch Wasser enthalten, werden nachbehandelt 73, verdichtet 72, und das verbleibende Restgas R' wieder in den Kreislauf der Verwertungsanlage 2 eingespeist.

Befinden sich der Verwertungs-Anlagenteil 2 und der Spitzenlast-Anlagenteil 7 am gleichen Ort, kann die Rückleitung des Restgases R' direkt erfolgen. In einer vorteilhaften Variante ist ein Zwischenspeicher 57 vorgesehen, wie in Figur 9 dargestellt. Wie oben stehend bereits ausgeführt kann der Anlagenteil 7 der erfindungsgemäss Kraftwerksanlage 1 von der Verwertungsanlage 2 getrennt angeordnet sein.

Die thermische oder elektrische Energie erzeugende Oxidationsreaktion erfolgt in der Antriebsvorrichtung 71 mit reinem Sauerstoff F anstatt mit Luft. Die Verwendung von Sauerstoff F anstatt Luft vermeidet zum einen aufgrund der Abwesenheit des Luftstickstoffs bei einer thermisch-chemischen Reaktion bei hohen Temperaturen die Bildung von Stickoxiden, vor allem aber verbleiben in den anfallenden Produktgase Z im Wesentlichen nur Kohlendioxid und Wasserdampf. Je nach Stöchiometrie der Reaktion können die anfallenden Gase auch gewisse Anteile an Kohlenmonoxid und unreagiertem Betriebsstoff enthalten. Diese können unproblematisch ebenfalls in den Kreislauf der Verwertungsanlage 2 einspeist werden.

Die Reaktionsprodukte Z der Energie erzeugenden Oxidationsreaktion sind im Wesentlichen gasförmig. Das entsprechende Produktgasgemisch wird nun verdichtet 72, um das Volumen zu reduzieren. Mit Hilfe eines Wärmetauschers 73 kann vor und/oder nach der Verdichtung das Produktgasgemisch Z abgekühlt werden. Wasser M' wird dabei auskondensiert und abgetrennt, wodurch im Restgas R' nur Kohlendioxid verbleibt, gegebenenfalls mit Anteilen an Kohlenmonoxid und unreagiertem Betriebsstoff. Das Restgas R' wird nun der ersten Stufe 3 der des ersten Anlagenteils 2 zugeführt, so dass sich ein geschlossener Stoffkreislauf für das Kohlendioxid ergibt. Alternativ kann das Restgas R' auch in die zweite Stufe 4 oder die dritte Stufe 5 geführt werden, was in Figur 9 durch gestrichelte Pfeile angedeutet ist.

So ist es möglich, dass in einer erfindungsgemässen Kraftwerksanlage 1 aus kohlenstoffhaltigen Substanzen A flüssige oder gasförmige Kohlenwasserstoffe und Kohlenwasserstoffderivate erzeugt werden, und das so resultierende hochwertige Betriebsstoffgemisch Q anschliessend in elektrische Energie 2 umgesetzt wird. Das produzierte Kohlendioxid wird rückgeführt und teilweise oder vollständig in der Verwertungsanlage 2 wieder in Betriebstoff Q umgesetzt. Auf diese Weise kann der effektive Kohlendioxidausstoss der Spitzenlast-Generatoranlage 7 sehr stark vermindert oder gar ganz vermieden werden.

Die Antriebsvorrichtung kann auch problemlos im Kombibetrieb mit Wasserstoff N als weiterem Betriebsstoff betrieben werden. In einem solchen Fall führt der Wasserstoffanteil zu einer Reduktion der anfallenden Restgasmenge R' nach dem Wärmetauscher/Kondensator und Verdichter, da bei der Oxidation von Wasserstoff mit Sauerstoff ohnehin nur Wasser anfällt.

Eine andere vorteilhafte Ausführungsform einer erfindungsgemässen Kraftwerksanlage 1 ist in Figur 10 gezeigt. Diese umfassend neben der Verwertungsanlage 2 sowohl einen Grundlastanlagenteil 6, als auch einen Spitzenlast-Anlagenteil 7.

Ist eine Antriebsvorrichtung 71 als Verbrennungskraftmaschine ausgelegt, so kann in einer vorteilhaften Variante einer solchen Antriebsvorrichtung Wasser als zusätzliches Expansionsmittel verwendet werden. Zu diesem Zweck wird nach der Zündung des Verbrennungsvorgangs, beispielsweise nach der Selbstzündung des verdichteten Treibstoff-Luft-Gemischs in einem Dieselmotor, eine bestimmte Menge Wasser in den Zylinder eingespritzt. Dieses Wasser, das vorzugsweise fein zerstäubt ist, wird anschliessend durch die Wärmeenergie der exothermen Oxidationsreaktion verdampft. Der daraus resultierende Gasdruck- bzw. Gasvolumenzuwachs aufgrund des Wasserdampfs trägt so zur Erzeugung der kinetischen Energie bei, wobei jedoch gleichzeitig die Temperatur des Gesamtgemischs an Verbrennungsabgasen und Wasserdampf sinkt. Dies ist jedoch unproblematisch oder sogar wünschenswert, weil aufgrund der höheren Energiedichte einer Reaktion mit reinem Sauerstoff wesentlich höhere Reaktionstemperaturen entstehen, was die thermodynamische Effizienz verbessert, aber auch die Teile einer Antriebsvorrichtung 71 stärker belasten kann.

Alternativ kann das Wasser auch als Dampf eingebracht werden. Ein gewisser Anteil an flüssigem Wasser kann zudem auch mit dem flüssigen Betriebsstoff vermischt zugeführt werden. Bei hohen Reaktionstemperaturen wirkt überhitzter Wasserdampf zudem als zusätzliches Oxidationsmittel neben dem Sauerstoff.

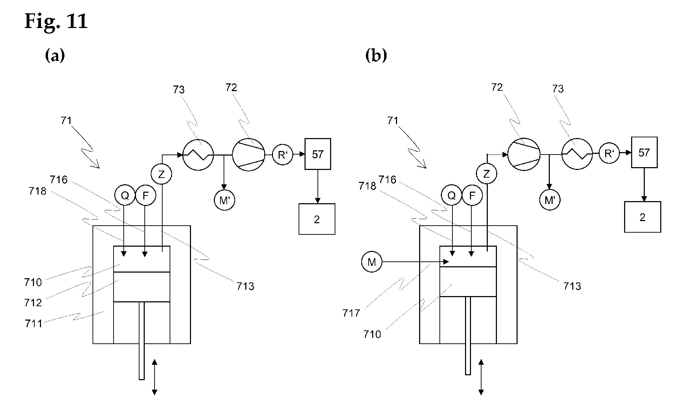

Nachfolgend wird in Figur 11 die Funktionsweise einer möglichen Antriebsvorrichtung 71 in einem dritten Anlagenteil einer erfindungsgemässen Kraftwerksanlage 1 am Beispiel einer Verbrennungskraftmaschine in Form eines Kolbenmotors genauer beschrieben und erläutert. Analog können als Verbrennungskraftmaschinen ausgestaltete Antriebsvorrichtungen 71 jedoch auch als Turbinen oder Wankel-Motoren ausgestaltet sein. Die heissen Verbrennungsgase werden entsprechend dem Funktionsprinzip des jeweiligen Typs einer Verbrennungskraftmaschine für die Leistung mechanischer Arbeit zum Beispiel zum Betrieb einer Generatoranlage verwendet, und dabei teilweise entspannt. Anschliessend verlässt das Produktgas Z die Brennkammer. So wird beispielsweise bei einer als Viertakt-Kolbenmotor ausgestalteten erfindungsgemässen Verbrennungskraftmaschine beim dritten Takt das Verbrennungsgasgemisch Z aus dem Zylinder ausgestossen, und anschliessend verdichtet und abgekühlt. Ebenfalls ist es möglich, eine erfindungsgemässe Antriebsvorrichtung 71 als Wärmekraftmaschine mit äusserer Verbrennung zu realisieren beispielsweise als Dampfmaschine bzw. Dampfturbine.

Eine mögliche Ausführungsform einer als Verbrennungskraftmaschine ausgestalteten Antriebsvorrichtung 71 ist schematisch in Figur 11 (a) dargestellt, am Beispiel eines Kolbenmotors mit einem Zylinder. Die dargestellte Verbrennungskraftmaschine 71 weist einen Zylinder 711 und einen darin beweglich angeordneten Kolben 712 auf, welche zusammen eine geschlossene Brennkammer 710 bilden. Mit einer lediglich schematisch dargestellten Zufuhrvorrichtung 716 wird in einem ersten Takt Sauerstoff F in die expandierende Brennkammer 710 eingebracht. Anschliessend wird in einem zweiten Takt der Sauerstoff F komprimiert, und am Ende des zweiten Takts mit einer Zufuhrvorrichtung 718 der Betriebsstoff Q in die Brennkammer 710 eingebracht und verbrannt. Beim darauffolgenden dritten Takt verrichten die expandierenden Verbrennungsgase Z mechanische Arbeit, und beim vierten Takt werden die teilweise entspannten Verbrennungsgase Z durch eine nicht näher dargestellte Entlüftungsvorrichtung 713 aus der Brennkammer 710 abgeführt.

Die heissen Produktgase Z, die im Wesentlichen nur aus Kohlendioxid und Wasserdampf bestehen, werden anschliessend in einem nachgeschalteten Wärmetauscher 73 abgekühlt. Dadurch wird das Volumen dieser Produktgase Z reduziert. Durch die Abkühlung kondensiert der Grossteil des Wassers M' aus und wird abgetrennt. Das verbleibende Restgas R', das im Wesentlichen nur noch aus Kohlendioxid und gegebenenfalls Restanteilen Kohlenmonoxid und unreagierten Betriebsstoffen besteht, wird in einem in Serie angeordneten Verdichter 72 komprimiert und in einem Druckspeicher 57 aufgefangen. Die Kondensationsstufe 73 vor der Verdichtung verringert dabei die unerwünschte Bildung von Kondenswassertröpfchen im Verdichter 72.

Die dargestellte Verbrennungskraftmaschine 71 weist keine Emissionen auf. Da die Vorrichtung nicht mit Luft oder ähnlichen Gasgemischen als Oxidationsmittel betrieben wird, können auch keine luftspezifischen Schadstoffe wie beispielsweise Stickoxide entstehen. Das bei der Verbrennung entstehende Wasser ist unproblematisch, und kann abgetrennt werden. Das Kohlendioxid wird als Restgas R' in den Kreislauf der Verwertungsanlage 2 geführt. Unverbrannte Anteile des Betriebsstoffes kondensieren entweder zusammen mit dem Wasser aus und werden abgetrennt, oder werden zusammen mit den Kohlendioxid verdichtet.

Die Produktgase Z aus der Antriebsvorrichtung 71 können auch ohne Kühlung direkt in die erste oder zweite Stufe geleitet werden.

Ist der Spitzenlast-Anlagenteil 7 räumlich getrennt, und ist eine direkte Rückleitung der Restgase R' nicht praktikabel, so können diese auch sehr stark verdichtet und unter hohem Druck in Druckspeichern 57 vom dritten Anlagenteil 7 zur restlichen Anlage zurücktransportiert werden.

Eine weitere mögliche Ausführungsform einer als Verbrennungskraftmaschine ausgestalteten Antriebsvorrichtung 71 ist schematisch in Figur 11(b) dargestellt. In dieser Variante wird durch eine lediglich schematisch dargestellte Zufuhrvorrichtung 717 Wasser M in die Brennkammer 710 eingebracht. Dies geschieht vorzugsweise so, dass während oder nach der Verbrennungsreaktion eine bestimmte Menge Wasser, flüssig oder dampfförmig, in die Brennkammer 710 eingespritzt und fein verteilt wird. Dieses Wasser wird durch die Verbrennungswärme erhitzt, wodurch das gesamte Gasvolumen in der Brennkammer 710 steigt, und damit auch der für die Leistung der mechanischen Arbeit zur Verfügung stehende Gasdruck bzw. Gasvolumen. Entsprechend kann dann bei gleichbleibender Leistung die Menge an Betriebsstoff gesenkt werden.

Alternativ oder zusätzlich kann Wasser M auch in den Produktgasstrom Z eingebracht werden, wenn dieser die Brennkammer 710 verlassen hat. Eine solche Variante hat den Vorteil, dass die Verbrennungsreaktion in der Brennkammer bei möglichst hohen Temperaturen effizient verlaufen kann, und gleichzeitig die resultierende Temperatur des Produktgasstromes so niedrig ist, dass die nachfolgenden Einrichtungen 73, 72 nicht zu sehr belastet werden.

Die Menge an Wasser und der Zeitpunkt des Einspritzens werden so mit der Zufuhr von Betriebsstoff Q und Sauerstoff F abgestimmt, dass die Verbrennungsreaktion effizient stattfinden kann. Vorteilhaft liegt die resultierende Temperatur während der Oxidationsreaktion im Wesentlichen so, dass ein möglichst hoher thermodynamischer Wirkungsgrad der Wärmekraftmaschine erreicht wird. Je grösser die Menge an verwendetem Wasser ist, desto geringer ist zudem der relative Anteil an Kohlendioxid in den Reaktionsgasen, was die nach der Auskondensation des Wassers M' verbleibende Restgasmenge R' reduziert.

In der in Figur 11(b) dargestellten Ausführungsform werden die Produktgase Z zuerst in einem Verdichter 72 komprimiert, bevor sie anschliessend im Wärmetauscher 73 abgekühlt werden. Diese Variante ist auch mit der Verbrennungskraftmaschine 71 ohne Wassereinspritzung aus Figur 11(a) kombinierbar, und umgekehrt, und kann allgemein für eine erfindungsgemässe Antriebsvorrichtung 71 verwendet werden.

Die für den Betrieb des Verdichters einer erfindungsgemässen Antriebsvorrichtung 71 notwendige Energie wird vorteilhaft durch die erfindungsgemässe Antriebsvorrichtung selber erzeugt. Als Folge davon sinkt der erreichbare Wirkungsgrad der erfindungsgemässen Antriebsvorrichtung, jedoch wird damit gleichzeitig die Emissionsfreiheit der genannten Antriebsvorrichtung erreicht. Zudem ist die erreichbare Leistung bei gleicher Motorendimensionierung grösser, was den Leistungsverlust wieder ausgleicht. Der Verdichter kann beispielsweise über ein geeignetes Getriebe direkt mit der Kurbelwelle einer Kolben-Verbrennungskraftmaschine betrieben werden.

Umfasst die erfindungsgemässe Antriebsvorrichtung 71 eine Turbine, so kann der Verdichter direkt auf der gleichen Welle sitzen. Die Produktgase können dann direkt anschliessend an den Expansionsvorgang kondensiert und der verbleibende Reststrom verdichtet werden.

In einer anderen Variante einer als Kolbenmotor ausgestalteten erfindungsgemässen Antriebsvorrichtung werden die Produktgase nach der Verbrennung beim dritten Takt innerhalb der Brennkammer bereits vorkomprimiert, und erst dann durch die Entlüftungsvorrichtung 713 abgelassen. Gegebenenfalls kann der nachgeschaltete Verdichter 72 auch weggelassen werden.

Eine solche Ausführungsform ist auch als Zweitakt-Variante möglich, weil die neue Beladung der Brennkammer mit Reaktionsgemisch (Betriebstoff Q, Sauerstoff F, Wasser M) in einer Antriebsvorrichtung sehr schnell erfolgen kann. In einem zweiten Aufwärtstakt werden die Verbrennungsgase vorkomprimiert, und gegen Ende des Takts aus der Brennkammer abgelassen. Der gasförmige Sauerstoff kann unter hohem Druck am Ende des Aufwärtstakts in die Brennkammer eingeblasen werden, da für eine vollständige Verbrennungsreaktion vergleichsweise wenig Sauerstoff benötigt wird, und Wasser als zusätzliches Expansionsmittel vorhanden ist. Der flüssige Betriebsstoff Q und das Wasser M als Expansionsmittel können ohnehin sehr schnell und unter hohem Druck in die Brennkammer 710 eingespritzt werden.

Der Energieverbrauch für den Verdichter 72 kann optimiert werden durch eine geeignete Kombination mit einem oder mehreren Wärmetauschern beziehungsweise Kühlelementen, in denen durch Abgabe von Wärmeenergie der Reaktionsgase an eine internes oder externe Wärmesenke das Gasvolumen reduziert werden kann.

Mit dem Wärmetauscher/Kondensator 73 kann Dampf erzeugt werden, welcher entweder zur Effizienzsteigerung der Dampfturbinenanlage 6 dienen kann, oder zur Gewinnung von Prozessdampf B für den Betrieb der Verwertungsanlage 2.

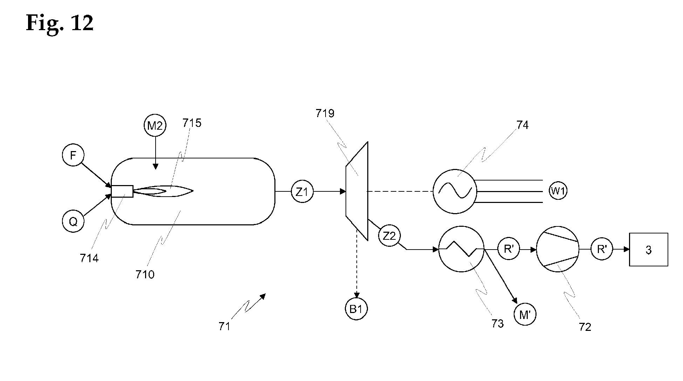

Figur 12 zeigt eine besonders vorteilhafte Ausführungsvariante einer Spitzenlast-Generatorstufe 7, mit einer Antriebsvorrichtung 71, die als kombinierte Gas/Dampfturbine ausgestaltet ist. In einer vorgeschalteten Brennkammer 710 wird Betriebstoff Q mit Sauerstoff F in einem Brenner 714 verbrannt, unter Bildung eines sehr heissen Verbrennungsgases. In die Brennkammer 710 wird Wasser eingebracht, vorzugsweise als überhitztes flüssiges Wasser mit einet Temperatur von beispielsweise 250 °C und einem Druck von 50 bar. Der resultierende Wasserdampf vermischt sich mit den Verbrennungsabgasen, so dass ein heisses (z.B. 600°C) Produktgas Z1 mit einem hohen Anteil an überhitztem Wasserdampf entsteht, welches aus der Brennkammer 710 austritt und in einer nachfolgenden Turbinenvorrichtung 719 in mechanische Arbeit umgesetzt wird, mit welcher wiederum eine Generatorvorrichtung 74 angetrieben wird. Je nach Ausgestaltung verhält sich das Gasgemisch in der Brennkammer isochor, so dass der Gasdruck steigt, oder isobar, so dass das Gasvolumen entsprechend ansteigt, oder sowohl das Volumen als auch der Druck steigen an. Entsprechend muss auch die nachfolgende Turbinenvorrichtung 719 ausgestaltet werden. Geeignete Turbinen 719 sind aus dem Stand der Technik bekannt, und verfügen meist über mehrere Stufen. In einer alternativen Variante kann nach einer Hochdruckstufe der Turbinenvorrichtung 719 teilentspannter Prozessdampf 1 abgezogen und anderweitig verwendet werden.

Das entspannte Produktgas Z2 wird in einen Kondensator/Ecomomizer 73 geleitet, wo das Wasser M' auskondensiert und abgetrennt wird. Das verbleibende Restgas R', welches im wesentlichen Kohlendioxid enthält, wird in einem Verdichter 72 komprimiert und in die erste Stufe 3 einer Verwertungsanlage 2 gefördert. Der Verdichter 72 wird vorteilhaft direkt über die Turbine 719 angetrieben.

Anstatt in die Brennkammer 710 kann das Wasser M2 auch erst anschliessend an die Brennkammer 710 mit dem Produktgasstrom Z1 gemischt werden, beispielsweise mittels einer Venturidüse.

In der Antriebsvorrichtung 71 werden die Menge Wasser M2 und die Menge an Brenngemisch Q, F und die weiteren wählbaren Parameter vorteilhaft so aufeinander abgestimmt, dass die nachfolgende Turbine eine möglichst hohe Energieausnutzung erreicht. Gleichzeitig soll der Anteil Wasser am Produktgasgemisch Z2 möglichst hoch sein. Zum einen wird so ein möglichst hoher Druckabfall des Gasgemischs auf dem Kondensator 73 erreicht, was die totale Druckdifferenz über die Turbine 719 erhöht, und somit deren Effizienz. Zum anderen verbleibt weniger Restgas R', das verdichtet 72 werden muss.

Ein weiterer Vorteil des Einbringens von Dampf in die Brennkammer ist der kühlende Effekt des Dampfs B. Die exotherme Oxidation des Brennstoffgemischs Q, F kann zu sehr hohen Temperaturen führen, von bis zu 1000 °C oder gar 2000 °C. Solche Temperaturen würden die Strukturen der Brennkammer 710 und der nachfolgenden Turbinenvorrichtung 719 sehr stark belasten. Der vergleichsweise kalte Wasserdampf wird vorzugsweise so in die Kammer eingebracht, dass er die Wände der Brennkammer 710 von der sehr heissen Flamme 715 abschirmt. Der Dampf kühlt schliesslich das gesamte Gasgemisch auf 600 °C bis 800°C ab, was die die thermische Belastung der Turbinenblätter senkt und entsprechend die Lebensdauer erhöht.

Neben den bereits erwähnten Aspekten unterscheidet sich die dargestellte Antriebsvorrichtung beispielsweise von einer herkömmlichen Gasturbine auch dadurch, dass der Brennkammer kein Verdichter vorgeschaltet ist. Dies erlaubt eine wesentlich einfachere Gestaltung der Brennkammer 710 als bei einer Gasturbine. Da die Betriebsstoffe Q mit reinem Sauerstoff F verbrannt werden, ist die erreichbare Energiedichte höher als bei Verwendung von Luft mit seinem reduzierten Sauerstoffanteil. Um dennoch die Menge an pro Zeiteinheit in die Brennkammer 710 einbringbarem Sauerstoff zu erhöhen, kann dieser vorab unter Druck gesetzt werden. Dir Turbinenvorrichtung 719 kann wie eine Dampfturbine gestaltet sein, da die Temperatur- und Druckbereiche des Produktgases Z1 im Wesentlichen dieselben sind.

Im Normalbetrieb einer erfindungsgemässen Kraftwerksanlage 1 bleibt die Antriebsvorrichtung 71 im Leerlauf. Eine geringe Menge an Dampf hält die Turbine 719 in Bewegung, während die Generatorvorrichtung keinen Strom produziert. Steigt nun kurzfristig der Strombedarf, wird Brennstoffgemisch F, Q in die Brennkammer 710 eingespritzt, und mit einer (nicht dargestellten) Zündvorrichtung gezündet. Gleichzeitig wird die Menge an eingespritztem Wasser M2 B erhöht. Die Turbine 719 fährt nun hoch, und der Generator 74 wird in Betrieb genommen.

Die Antriebsvorrichtung 71 kann auch dauerhaft in Betrieb sein, beispielsweise bei 10% bis 50% der Leistung der Grundlast-Generatoranlage 6. Bei Erhöhung des Strombedarfs kann dann die Anlage 7 in kürzester Zeit auf Maximalleistung gebracht werden, beispielsweise 500% der Leistung der Grundlast-Generatoranlage 6. Eine erfindungsgemässe Kraftwerksanlage 1 kann so sehr dynamisch die Gesamtleistung über einen breiten Bereich anpassen. Ein Spitzenlast-Anlagenteil kann auch mehrere Brennkammern 710 und/oder Turbinenvorrichtungen 719 aufweisen.

### Bezugszeichenliste

- 1: Kraftwerksanlage
- 2: erster Anlagenteil, Verwertungsanlage
- 211, 212, 213: Verdichter
- 22: Zyklonabscheider
- 231,232,233: Wärmetauscher
- 231: Wärmetauscher, Überhitzer des Dampfkreislaufs des zweiten Anlagenteils
- 241,242: Druckschleuse
- 25: Druckreduzierung
- 261,262,263,264: Absperrventil
- 27: Rückschlagventil
- 28: Silo, Vorratsbehälter
- 29: Schlackelager
- 3: erste Stufe
- 30: Pyrolyse-Reaktor, erster Druckreaktor
- 31: Druckkörper
- 32: Vorschubrost
- 331,332: Heizungsvorrichtung
- 4: zweite Stufe
- 40: Vergasungs-Reaktor, zweiter Druckreaktor
- 41: Druckkörper
- 42: Vorschubrost
- 5: dritte Stufe
- 50: Fischer-Tropsch-Reaktor, Synthese-Reaktor
- 51: Kühlung Synthesestufe, Boiler des Dampfkreislaufs des zweiten Anlagenteils
- 52: gemeinsamer Druckreaktor erste und zweite Stufe
- 53: erste Kammer
- 54: zweite Kammer
- 55: Trennwand
- 56: Betriebsstoffspeicher
- 57: Produktgasspeicher
- 6: zweiter Anlagenteil, Grundlast-Generatoranlage, Grundlast-Anlagenteil
- 61: Antriebsvorrichtung
- 619: Dampfturbine
- 62: Pumpe
- 63: Kondensator, Ecomomizer
- 64: Generatorvorrichtung
- 65: externer Kühlkreislauf
- 7: dritter Anlagenteil, Spitzenlast-Generator-Anlage, Spitzenlast-Anlagenteil
- 71: Antriebsvorrichtung
- 710: Brennkammer
- 711: Zylinder
- 712: Kolben
- 713: Entlüftungsvorrichtung
- 714: Brenner
- 715: Flamme
- 716: Zufuhrvorrichtung für Sauerstoff
- 717: Zufuhrvorrichtung für Wasser
- 718: Zufuhrvorrichtung für Betriebsstoff
- 719: Turbine
- 72: Verdichter
- 73: Kondensator/Economizer
- 74: Generatorvorrichtung
- 75: externer Kühlkreislauf
- a: Wärmeinhalt
- b: thermische Leistung eines herkömmlichen Kraftwerks
- c, c': Grundlast-Leistung
- d: effektive thermische Leistung eines erfindungsgemässen Kraftwerks
- e, e': Gesamt-Leistung
- f: Grundlast-Leistung des Grundlast-Anlagenteils
- g: Betriebsstoff-Produktionsleistung der Verwertungsanlage
- A, A': kohlenstoffhaltiges Ausgangsmaterial
- B, B1: Prozessdampf
- C, C2, C3,: Dampf im Turbinenkreislauf
- D: Pyrolyse-Koks
- E: Pyrolyse-Gas
- F: Sauerstoff, Oxidationsmittel
- G: zusätzliche Brennstoffe
- H: Schlacke, Asche, Reststoffe
- J: Reststoffe
- K: Reststaub
- L: Graphit, Aktivkohle, kohlehaltige Reststoffe
- M, M', M1, M2: Wasser, Speisewasser, Prozesswasser, Kondensat
- N: Wasserstoffgas
- P: Katalysator
- Q: Produkte der Synthese-Stufe, Betriebsstoff
- R, R1, R2, R3: Rücklaufgas
- R': Restgas
- S, S1, S2: Synthesegas
- T: Kohlendioxid
- U: schwerflüchtige Anteile des Pyrolysegases
- W1, W2: elektrische bzw. mechanische Energie
- Z, Z1, Z2: Produktgase

## Patentansprüche

1. Anlage (1) zur Erzeugung von elektrischer und/oder mechanischer Energie (W2) durch thermisch-chemische Verwertung von kohlenstoffhaltigen Substanzen (A), mit einem ersten Anlagenteil (2) zur Verwertung der kohlenstoffhaltigen Substanzen (A), und einem weiteren Anlagenteil (7) zur Erzeugung von elektrischer und/oder mechanischer Energie (W2), **dadurch gekennzeichnet, dass**
der erste Anlagenteil (2) eine erste Stufe (3) zur Durchführung einer Pyrolyse der kohlenstoffhaltigen Substanzen (A) zu Pyrolysekoks (D) und Pyrolysegas (E), eine zweite Stufe (4) zur Durchführung einer Vergasung des Pyrolysekokses (D) zu Synthesegas (S) und Reststoffen (H, K) und eine dritte Stufe (5) zur Durchführung einer Umwandlung des Synthesegases (S) in kohlenstoffhaltige Betriebsstoffe (Q) umfasst,
wobei alle drei Stufen (3, 4, 5) des ersten Anlagenteils (2) druckfest geschlossen sind und einen im Wesentlichen geschlossenen Kreislauf bilden; eine Transportleitung für das Pyrolysegas (E) den ersten Anlagenteil (3) druckfest mit dem zweiten Anlagenteil (4) und/oder mit dem dritten Anlagenteil (5) verbindet; eine Transportleitung für das Synthesegas (S) den zweiten Anlagenteil (4) druckfest mit dem dritten Anlagenteil (5) und/oder mit dem ersten Anlagenteil (3) verbindet; und eine Transportleitung für das Rücklaufgas (R) den dritten Anlagenteil (5) druckfest mit dem ersten Anlagenteil (3) und/oder mit dem zweiten Anlagenteil (4) verbindet;
und dass in dem weiteren Anlagenteil (7) mindestens eine Antriebsvorrichtung (71) vorgesehen ist zur Produktion (74) von elektrischer und/oder mechanischer Energie (W2) aus den kohlenstoffhaltigen Betriebsstoffen (Q).

2. Anlage nach Anspruch 1, **dadurch gekennzeichnet, dass** die Antriebsvorrichtung (71) die zum Betrieb notwendige Energie aus der Oxidation der kohlenstoffhaltigen Betriebsstoffe (Q) zu einem Produktgas (Z) im wesentlichen bestehend aus Kohlendioxid und Wasser bezieht, und eine Vorrichtung zur Verdichtung (72) und/oder Kondensation (73) des Produktgases (Z) umfasst.

3. Anlage nach Anspruch 2, **dadurch gekennzeichnet, dass** die Antriebsvorrichtung (71) mit reinem Sauerstoff (F) als Oxidationsmittel betreibbar ist.

4. Anlage nach Anspruch 2 oder 3, **gekennzeichnet durch** einen Wärmetauscher (73) zur Abkühlung des Produktgasstromes (Z) vor und/oder nach der Vorrichtung (72) zur Verdichtung und/oder Kondensation des Produktgases (Z).

5. Anlage nach einem der Ansprüche 2 bis 4, **gekennzeichnet durch** eine Vorrichtung zur Kondensation und/oder Abscheidung von Wasser (M') aus dem Produktgas (Z).

6. Anlage nach einem der Ansprüche 2 bis 5, **dadurch gekennzeichnet, dass** ein Speicher (57) zum Auffangen des Produktgases (Z) bzw. Restgases (R') nach Verdichtung und/oder Kondensation des Produktgases (Z) vorgesehen ist.

7. Anlage nach einem der vorangehenden Ansprüche, **dadurch gekennzeichnet, dass** die Antriebsvorrichtung (71) eine Verbrennungskraftmaschine ist, mit mindestens einer Brennkammer (710) zur Verbrennung von flüssigem oder gasförmigem Betriebsstoff (Q) mit Sauerstoff (F), mit Mitteln (710, 719) zur Umsetzung des entstehenden Gasdrucks bzw. Gasvolumens in mechanische Arbeit, mit einer Zufuhrvorrichtung (716) zum Einbringen von Sauerstoff (F) in die Brennkammer (710), und mit einer Entlüftungsvorrichtung (713) zur Entfernung der Produktgase (Z) aus der Brennkammer (710).

8. Anlage nach Anspruch 7, **gekennzeichnet durch** eine Zufuhrvorrichtung (717) zum Einbringen von Wasser (M) und/oder Wasserdampf (B) in die Brennkammer (710) und/oder in den Produktgasstrom (Z) nach dem Austritt aus der Brennkammer (710).

9. Anlage nach einem der vorangehenden Ansprüche, **dadurch gekennzeichnet, dass** im Kreislauf des ersten Anlagenteils (2) mindestens ein Wärmetauscher (231, 232, 51) zur Erhitzung von Wasserdampf (C1, C2) und/oder Erzeugung von Wasserdampf (B, B1) vorgesehen ist, und dass in dem weiteren Anlagenteil (6) eine weitere Antriebsvorrichtung (61), bevorzugt eine Dampfturbine (619), vorgesehen ist zur Produktion (64) von elektrischer und/oder mechanischer Energie (W1) aus erhitztem Wasserdampf (C2).

10. Anlage (1) zur Erzeugung von elektrischer und/oder mechanischer Energie (W1) durch thermisch-chemische Verwertung von kohlenstoffhaltigen Substanzen (A), mit einem ersten Anlagenteil (2) zur Verwertung der kohlenstoffhaltigen Substanzen (A), und einem weiteren Anlagenteil (6) zur Erzeugung von elektrischer und/oder mechanischer Energie (W1), **dadurch gekennzeichnet, dass**
der erste Anlagenteil (2) eine erste Stufe (3) zur Durchführung einer Pyrolyse der kohlenstoffhaltigen Substanzen (A) zu Pyrolysekoks (D) und Pyrolysegas (E), eine zweite Stufe (4) zur Durchführung einer Vergasung des Pyrolysekokses (D) zu Synthesegas (S) und Reststoffen (H, K) und eine dritte Stufe (5) zur Durchführung einer Umwandlung des Synthesegases (S) in kohlenstoffhaltige Betriebsstoffe (Q) umfasst,
wobei alle drei Stufen (3, 4, 5) des ersten Anlagenteils (2) druckfest geschlossen sind und einen im Wesentlichen geschlossenen Kreislauf bilden; eine Transportleitung für das Pyrolysegas (E) den ersten Anlagenteil (3) druckfest mit dem zweiten Anlagenteil (4) und/oder mit dem dritten Anlagenteil (5) verbindet; eine Transportleitung für das Synthesegas (S) den zweiten Anlagenteil (4) druckfest mit dem dritten Anlagenteil (5) und/oder mit dem ersten Anlagenteil (3) verbindet; und eine Transportleitung für das Rücklaufgas (R) den dritten Anlagenteil (5) druckfest mit dem ersten Anlagenteil (3) und/oder mit dem zweiten Anlagenteil (4) verbindet;
und wobei im Kreislauf des ersten Anlagenteils (2) mindestens ein Wärmetauscher (231, 232, 51) zur Erhitzung von Wasserdampf (C1, C2) und/oder Erzeugung von Wasserdampf (B, B1) vorgesehen ist,
und dass in dem weiteren Anlagenteil (6) eine Antriebsvorrichtung (61), bevorzugt eine Dampfturbine (619), vorgesehen ist zur Erzeugung (64) von elektrischer und/oder mechanischer Energie (W1) aus dem erhitzten Wasserdampf (C2).

11. Verfahren zur Erzeugung elektrischer und/oder mechanischer Energie (W2) durch thermisch-chemische Verwertung von kohlenstoffhaltigen Substanzen (A),
bei welchem in einer ersten Stufe (3) die kohlenstoffhaltigen Substanzen (A) zugeführt und pyrolysiert werden, wobei Pyrolysekoks (D) und Pyrolysegas (E) entstehen; in einer zweiten Stufe (4) der Pyrolysekoks (D) aus der ersten Stufe (3) vergast wird, wobei Synthesegas (S) entsteht, und Schlacke und andere Reststoffe (H, K) übrig bleiben und abgeführt werden; und in einer dritten Stufe (5) das Synthesegas (S) aus der zweiten Stufe (4) in kohlenstoffhaltige Betriebsstoffe (Q) umgewandelt wird, und diese Betriebsstoffe (Q) abgeführt werden; wobei überschüssiges Rücklaufgas (R) aus der dritten Stufe (5) in die erste Stufe (3) und/oder die zweite Stufe (4) geleitet wird, und wobei die drei Stufen (3, 4, 5) einen geschlossenen Kreislauf bilden; und
bei dem durch Oxidation der kohlenstoffhaltigen Betriebsstoffe (Q) aus der dritten Stufe (5) zu einem Produktgas (Z) im Wesentlichen bestehend aus Kohlendioxid und Wasser elektrische und/oder mechanische Energie (W2) erzeugt (71, 74) wird.

12. Verfahren nach Anspruch 11, **dadurch gekennzeichnet, dass** als Oxidationsmittel reiner Sauerstoff (F) verwendet wird.

13. Verfahren nach Anspruch 11 oder 12, **dadurch gekennzeichnet, dass** aus den Produktgasen (Z) Wasser auskondensiert und/oder abgeschieden wird.

14. Verfahren nach einem der Ansprüche 11 bis 13, **dadurch gekennzeichnet, dass** ein mindestens ein Teil der Produktgase (Z) wieder in die erste Stufe (3) und/oder die zweite Stufe (4) und/oder die dritte Stufe (5) eingespeist wird.

15. Verfahren zur Erzeugung von elektrischer und/oder mechanischer Energie (W1) durch thermisch-chemische Verwertung von kohlenstoffhaltigen Substanzen (A),
bei welchem in einer ersten Stufe (3) die kohlenstoffhaltigen Substanzen (A) zugeführt und pyrolysiert werden, wobei Pyrolysekoks (D) und Pyrolysegas (E) entstehen; in einer zweiten Stufe (4) der Pyrolysekoks (D) aus der ersten Stufe (3) vergast wird, wobei Synthesegas (S) entsteht, und Schlacke und andere Reststoffe (H, K) übrig bleiben und abgeführt werden; und in einer dritten Stufe (5) das Synthesegas (S) aus der zweiten Stufe (4) in kohlenstoffhaltige Betriebsstoffe (Q) umgewandelt wird, und diese Betriebsstoffe (Q) abgeführt werden; wobei überschüssiges Rücklaufgas (R) aus der dritten Stufe (5) in die erste Stufe (3) und/oder die zweite Stufe (4) geleitet wird, und wobei die drei Stufen (3, 4, 5) einen geschlossenen Kreislauf bilden; und
bei welchem in einem Wärmetauscher (231, 233) das Synthesegas (S) abgekühlt wird, wobei überhitzter Wasserdampf (B, B1, C1, C2) entsteht, aus welchem mit einer Wärmekraftmaschine (619) elektrische und/oder mechanische Energie (W1) produziert wird.
